# DEMANDE DE BREVET EUROPEEN

(11) **EP 2 065 024 A2**
(43) Date de publication de la demande: **03.06.2009**
(21) Numéro de dépôt: 08170204.5
(22) Date de dépôt: 28.11.2008
(51) Int. Cl.: A61K 8/37, A61K 8/81, A61K 8/92, A61Q 5/02, A61Q 5/06

(54) **Composition de coiffage comprenant des copolymères (meth)acryliques et au moins une huile**

(30) Priorité: 30.11.2007 FR 0759473
(71) Demandeur: L'Oreal, 75008 Paris (FR)
(72) Inventeur: Decoster, Sandrine, 95210 Saint-Gratien (FR); Proust, Jean-Mary, 78410 Aubergenville (FR)
(74) Mandataire: Casalonga, Axel

(57) **Abrégé**

L'invention concerne une composition de coiffage repositionnable comprenant, dans un véhicule cosmétiquement acceptable,
- une ou plusieurs huiles non-siliconées, et
- un ou plusieurs copolymères (méth)acryliques, comprenant :
(a) des motifs issus d'un ou plusieurs monomères choisis parmi les monomères de (méth)acrylate de butyle, le ou les monomères (a) représentant de 15 à 50 % en poids par rapport à la quantité totale de monomères,
(b) des motifs issus d'un ou plusieurs monomères choisis parmi les monomères de (méth)acrylate d'hydroxyalkyle et
(c) des motifs éventuels issus d'un ou plusieurs monomères copolymérisables autres que lesdits monomères (a) et (b), et de préférence une composition rincée.

## Description

La présente invention concerne une composition de coiffage repositionnable qui est de préférence une composition rincée.

La fixation de la coiffure est un élément important du coiffage, et implique la création d'une forme ou le maintien d'une forme qui a déjà été réalisée. Conformément à l'invention, le terme « composition de coiffage » concerne tout type de composition capillaire pouvant être utilisé pour effectuer le coiffage.

Les compositions de coiffage les plus courantes sur le marché des produits cosmétiques pour la mise en forme et/ou le maintien de la coiffure sont des compositions de spray comprenant une solution, habituellement à base d'alcool ou d'eau, et un ou plusieurs composés, généralement des résines polymères. L'une des fonctions de résines polymères est de former des liens entre les cheveux. Ces matières, également appelées fixateurs, se trouvent souvent en mélange avec différents adjuvants cosmétiques. Cette solution est généralement conditionnée soit dans un récipient aérosol approprié, qui est pressurisé à l'aide d'un propulseur, soit dans un flacon pompe.

De nombreuses compositions de coiffage existant à ce jour présentent le même inconvénient : elles ne sont pas destinées à permettre à la coiffure d'être modifiée ultérieurement en une forme recherchée, qui est autre que celle initialement formée, sans recommencer de nouveau les opérations de coiffage et de fixation. Par ailleurs, sous différents types de stress, la coiffure a tendance à prendre une forme permanente indésirable qui ne peut pas être facilement modifiée. De plus, au cours du procédé de coiffage, on peut rechercher les effets bénéfiques du conditionnement des cheveux, tels qu'une facilité de peignage et l'aspect doux au toucher des cheveux.

Il existe également des compositions rincées permettant un effet repositionnable. On entend par composition de coiffage « repositionnable » une composition de coiffage donnant un coiffage qui peut être restauré ou modifié sans appliquer à nouveau une matière ou de la chaleur. Par exemple, pour restaurer ou modifier la coiffure dans le cas « d'alourdissement » ou de perte de mise en pli (décoiffage), on n'a besoin d'aucune nouvelle matière telle que l'eau ou une forme quelconque d'agent de fixation, ni de chaleur supplémentaire. L'efficacité de la composition peut être durable conduisant à un effet coiffant durable.

Cependant, ces compositions de coiffage repositionnable ne permettent généralement pas d'obtenir un effet de recoiffage aussi prononcé que celui d'une composition non-rincée. En particulier, l'effet obtenu à l'aide d'une composition rincée se traduit généralement par un manque de tenue le jour même de l'application et dans le temps. De telles compositions sont décrites par exemple dans la demande de brevet EP-A-1 321 130.

Le but de la présente invention est l'obtention d'une composition de coiffage repositionnable, de préférence rincée, conférant une facilité de coiffage améliorée dès l'application, dont l'effet de recoiffage est satisfaisant et dont les propriétés cosmétiques, notamment au niveau de la douceur, du lissage, de la brillance et de la facilité de démêlage des cheveux sont améliorées.

Ce but est atteint par la présente invention, qui a pour objet une composition de coiffage repositionnable, de préférence rincée, comprenant, dans un véhicule cosmétiquement acceptable, (i) un ou plusieurs copolymères (méth)acryliques comprenant :
(a) des motifs issus d'un ou plusieurs monomères choisis parmi les monomères de (méth)acrylate de butyle, le ou les monomères (a) représentant de 15 à 50 % en poids par rapport à la quantité totale de monomères,
(b) des motifs issus d'un ou plusieurs monomères choisis parmi les monomères de (méth)acrylate d'hydroxyalkyle, et éventuellement
(c) des motifs issus d'un ou plusieurs monomères copolymérisables autres que lesdits monomères (a) et (b), et

### (ii) une ou plusieurs huiles non-siliconées.

Un autre objet de l'invention est un procédé de traitement cosmétique des cheveux, comprenant l'application sur les cheveux, avant leur mise en forme, d'une composition comprenant une ou plusieurs huiles non-siliconées et un ou plusieurs copolymères (méth)acryliques tels que décrits ci-dessus.

Un autre objet de l'invention est un procédé de traitement des cheveux, comprenant : (1) l'application sur les cheveux, avant la mise en forme initiale de la coiffure, d'une composition comprenant une ou plusieurs huiles non-siliconées et un ou plusieurs copolymères (méth)acryliques tels que décrits ci-dessus, ladite composition fournissant un effet repositionnable et étant de préférence une composition rincée telle qu'un shampooing ou un après-shampooing, puis (2) la mise en forme de la coiffure au moins une fois, sans avoir besoin de composition supplémentaire ou de chaleur supplémentaire.

L'invention concerne aussi l'utilisation de la composition selon l'invention comme shampooing ou après-shampooing.

Le copolymère (méth)acrylique utilisé selon l'invention est décrit dans EP-A-1 321 130.

Le terme « (méth)acrylate » englobe aussi bien le terme acrylate que le terme méthacrylate. De même, le terme « acide (méth)acrylique » englobe aussi bien l'acide acrylique que l'acide méthacrylique. On entend par « composition rincée » toute composition qui est formulée pour être rincée immédiatement ou après un temps de pose généralement inférieur à 30 minutes, de préférence inférieur à 10 minutes, après application sur les cheveux.

Les monomères (a) utilisables pour la préparation du copolymère (méth)acrylique (i) utilisé dans la composition selon l'invention sont les monomères de (méth)acrylates de butyle tels que par exemple les (méth)acrylates de n-butyle, les (méth)acrylates de t-butyle, les (méth)acrylates d'isobutyle, les (méth)acrylates de 2-décylbutyle et les (méth)acrylates de 2-méthylbutyle.

De préférence, les monomères (a) sont choisis parmi l'acrylate de n-butyle.

Les monomères (a) représentent de 15 à 50 % en poids, mieux de 30 à 40 % en poids, par rapport à la quantité totale de monomères utilisés.

Les monomères (b) utilisables pour la préparation du copolymère (méth)acrylique (i) utilisé dans la composition selon l'invention sont choisis parmi les monomères de (méth)acrylate d'hydroxy(C₁-C₄)alkyle, tels que par exemple les (méth)acrylates de 2-hydroxyéthyle, les (méth)acrylates d'hydroxypropyle, les (méth)acrylates de 2,3-dihydroxypropyle, les (méth)acrylates de 4-hydroxybutyle, et les (méth)acrylates de 2-hydroxypropyle.

De préférence, les monomères (b) sont choisis parmi les monomères d'acrylate de 2-hydroxyéthyle, de méthacrylate de 2-hydroxyéthyle et d'acrylate d'hydroxypropyle et plus particulièrement le méthacrylate de 2-hydroxyéthyle.

Les monomères (b) représentent généralement de 2 à 50 % en poids, de préférence de 2 à 25 % en poids, mieux de 2 à 10% en poids, par rapport à la quantité totale de monomères utilisés.

Selon un autre mode de réalisation distinct, les monomères (b) constituent de 10 à 50% en poids, de préférence de 10 à 30 % en poids, par rapport à la quantité totale de monomères utilisés.

Les monomères (c) éventuellement utilisables pour la préparation du copolymère (méth)acrylique (i) utilisé dans la composition selon l'invention peut être choisi parmi (i') les monomères de (méth)acrylate d'alkyle, de préférence les (méth)acrylates d'alkyle en C₁-C₂₄, (ii') les monomères polaires et (iii') les monomères à insaturation éthylénique polymérisables par voie radicalaire libre.

Les monomères de (méth)acrylate d'alkyle (i') peuvent inclure par exemple les (méth)acrylates de méthyle, les (méth)acrylates d'isobornyle, les (méth)acrylates d'éthyle, les (méth)acrylates d'iso-octyle, les (méth)acrylates de 2-éthylhexyle, les (méth)acrylates de lauryle, les (méth)acrylates d'octa-décyle, et des mélanges de ceux-ci. Dans un autre mode de mise en oeuvre, les motifs issus des monomères de (méth)acrylate de 2-éthylhexyle constituent de 30 à 80 % en poids de la quantité totale de monomères utilisés.

Les monomères polaires (ii') utiles incluent l'acide (méth)acrylique, l'acide itaconique, la N-vinylpyrrolidone, le N-vinylcaprolactame, les (méth)acrylamides substitués (tels que les N,N-diméthyl(méth)acrylamides et le N-octyl(méth)acrylamide), le (méth)acrylate de diméthylaminoéthyle, le (méth)acrylonitrile, le (méth)acrylate de 2-carboxyéthyle, l'anhydride maléique, et des mélanges de ceux-ci. Un autre exemple de monomères polaires (ii') utiles est le monoacrylate de méthoxypolyéthylèneglycol 550 disponible auprès de Sartomer Co. sous le nom commercial CD553.

Les monomères utiles à insaturation éthylénique polymérisables par voie radicalaire libre (iii') incluent le styrène et des esters de vinyle en C₁-C₄ tels que l'acétate de vinyle, le propionate de vinyle, et des mélanges de ceux-ci. Dans encore un autre mode de mise en oeuvre, les motifs issus de monomères à insaturation éthylénique polymérisable par voie radicalaire copolymérisable (iii') sont présents dans une concentration allant jusqu'à 30 % en poids par rapport au poids total de tous les monomères.

De préférence, les monomères (c) éventuellement utilisables pour la préparation du copolymère (méth)acrylique (i) utilisé dans la composition selon l'invention est le (méth)acrylate de 2-éthylhexyle.

Les monomères (c) éventuellement utilisables pour la préparation du copolymère (méth)acrylique (i) utilisé dans la composition selon l'invention constituent par exemple jusqu'à 80 % en poids, de préférence de 30 à 80 % en poids, mieux encore de 50 à 70 % en poids, par rapport à la quantité totale de monomères utilisés.

Selon un autre mode de réalisation, les monomères (c) peuvent constituer jusqu'à 50 % en poids, de préférence jusqu'à 30 % en poids, par rapport à la quantité totale de monomères utilisés.

De préférence, le ou les copolymères (méth)acryliques (i) comprennent uniquement les monomères a), b) et c).

De préférence, le ou les copolymères (méth)acryliques (i) sont non siliconés, c'est-à-dire qu'ils ne comprennent pas dans leur structure d'atome de silicium.

Le ou les copolymères (méth)acryliques (i) utilisés dans la composition selon l'invention sont préparés à partir d'un mélange de monomères pouvant contenir un ou plusieurs agents de réticulation polyfonctionnels.

Au sens de la présente invention, on entend par « agent de réticulation polyfonctionnel », un agent de réticulation ayant une fonctionnalité moyenne supérieure à 1, par exemple supérieure à 1,8 et de préférence supérieure à 2,0. Cependant, la fonctionnalité moyenne est inférieure à 6, par exemple inférieure à 4, et de préférence égale ou inférieure à 3.

Des exemples d'agents de réticulation incluent, mais sans s'y limiter, ceux choisis parmi le divinylbenzène, les diacrylates d'alkyle (tels que ceux choisis parmi le diacrylate de 1,2-éthylèneglycol, le diacrylate de 1,4-butanediol, le diacrylate de 1,6-hexanediol, le diacrylate de 1,8-octanediol et le diacrylate de 1,12-dodécanediol), les triacrylates d'alkyle et les tétra-acrylates d'alkyle (tels que le triacrylate de triméthylolpropane et le tétra-acrylate de pentaérythritol), les cétones aromatiques à insaturation monoéthylénique (telles que la 4-acryloxybenzophénone), les aziridine-amides multifonctionnels (tels que la 1,1'-(1,3-phénylènedicarbonyl)bis[2-méthylaziridine], la 2,2,4-triméthyladipoylbis[2-éthylaziridine], la 1,1'-azélaoylbis[2-méthylaziridine] et la 2,4,6-tris(2-éthyl-1-aziridinyl)-1,3,5-triazine), les réticulants à ions métalliques (tels que le cuivre, le zinc, le zirconium et le chrome) et des mélanges de ceux-ci. Dans un mode de mise en oeuvre, les réticulants à ions métalliques sont choisis parmi les esters chélatés d'acide ortho-titanique vendus sous la désignation commerciale TYZOR et disponibles sur le marché auprès de E.I. du Pont de Nemours Co. Dans un autre mode de mise en oeuvre, le TYZOR est le TYZOR AA, qui est l'acétylacétonate de titane.

De préférence, l'agent de réticulation est le diacrylate de 1,6-hexanediol.

Les agents de réticulation, lorsqu'ils sont utilisés, peuvent représenter jusqu'à 10 parties en poids, typiquement de 0,1 à 2 parties en poids du mélange copolymérisable total sur la base de 100 parties en poids des monomères cités dans (a), (b) et (c), lorsqu'ils sont présents.

Le ou les copolymères (méth)acryliques (i) utilisés dans la composition selon l'invention peuvent être éventuellement obtenus avec un ou plusieurs amorceurs hydrosolubles et/ou oléo-solubles, qui sont utilisés pour obtenir des copolymères (méth)acryliques sous forme d'émulsions.

De tels amorceurs, par exposition à la chaleur, génèrent des radicaux libres qui amorcent la (co)polymérisation des monomères de (méth)acrylate de butyle, des monomères de (méth)acrylate d'hydroxyalkyle et les composants éventuels de comonomère et d'agent de réticulation.

Selon un mode de mise en oeuvre, on utilise un ou plusieurs amorceurs hydrosolubles. Par « amorceur hydrosoluble », on entend au sens de la présente invention, des amorceurs ayant une solubilité dans l'eau mesurée à 25°C au moins égale à 0,1 gramme/litre (g/L) (obtention d'une solution macroscopiquement isotrope et transparente, colorée ou non). Cette solubilité est de préférence supérieure ou égale à 1 g/L.

Les amorceurs hydrosolubles convenables incluent, mais sans s'y limiter, ceux choisis parmi le persulfate de potassium, le persulfate d'ammonium, le persulfate de sodium, et des mélanges de ceux-ci ; les amorceurs oxydo-réducteurs tels que le produit de réaction des persulfates cités ci-dessus et des agents réducteurs tels que ceux choisis parmi le métabisulfite de sodium et le bisulfite de sodium ; et l'acide 4,4'-azobis(4-cyanopentanoïque) et ses sels solubles (par ex. de sodium, de potassium). Dans un autre mode de mise en oeuvre, l'amorceur hydrosoluble est le persulfate de potassium.

De manière alternative, les amorceurs oléo-solubles convenables incluent, mais sans s'y limiter, ceux choisis parmi les composés azoïques tels que VAZO 64 (1e 2,2'-azobis(isobutyronitrile) et VAZO 52 (le 2,2'-azobis(2,4-diméthylpentanenitrile)), tous deux disponibles auprès de E.I. du Pont de Numours Co. ; et les peroxydes tels que le peroxyde de benzoyle, le peroxyde de lauroyle et des mélanges de ceux-ci. Dans un mode de mise en oeuvre, l'amorceur thermique oléo-soluble est le 2,2'-azobis(isobutyronitrile).

Lorsqu'il(s) est (sont) utilisé(s), l'(les) amorceur(s) peut (peuvent) représenter de 0,05 à 1 partie en poids, également de 0,1 à 0,5 partie en poids sur la base de 100 parties en poids du mélange copolymérisable total.

Dans un autre mode de mise en oeuvre, le ou les copolymères (méth)acryliques (i) utilisés dans la composition selon l'invention peuvent être éventuellement obtenus avec un ou plusieurs agents de transfert de chaîne.

Des exemples d'agents de transfert de chaîne utiles incluent, mais sans s'y limiter, ceux choisis parmi le tétrabromure de carbone, les alcools, les mercaptans, et des mélanges de ceux-ci.

De manière particulièrement préférée, l'agent de transfert de chaîne est choisi parmi l'iso-octylthioglycolate et le tétrabromure de carbone.

Le mélange copolymérisable peut en outre comprendre jusqu'à 0,5 partie en poids d'un ou plusieurs agents de transfert de chaîne, typiquement de 0,01 % en poids à 0,5 partie en poids, s'ils sont utilisés, également de 0,05 partie en poids à 0,2 partie en poids, sur la base de 100 parties en poids du mélange copolymérisable total.

Plus préférentiellement, le ou les copolymères (méth)acryliques (i) utilisés dans la composition selon l'invention comprennent :
- (a) de 30 à 40 % en poids de motifs issus d'un ou plusieurs monomères choisis parmi les monomères d'acrylate de n-butyle,
- (b) de 2 à 10 % en poids de motifs issus d'un ou plusieurs monomères choisis parmi les monomères de méthacrylate de 2-hydroxyéthyle, et
- (c) de 50 à 70 % en poids de motifs issus d'un monomère d'acrylate de 2-éthylhexyle.

De manière encore plus préférée, le copolymère (méth)acrylique (i) utilisé dans la composition selon l'invention comprend :
- (a) 35 % en poids de motifs issus d'un monomère d'acrylate de butyle,
- (b) 5 % en poids de motifs issus d'un monomère de méthacrylate de 2-hydroxyéthyle,
- (c) 60 % en poids de motifs issus d'un monomère de d'acrylate de 2-éthylhexyle.

Un tel produit est proposé par la société 3M sous l'appellation CPC-00960 ACP 45 Acrylates Copolymer.

Les pourcentages en poids des motifs (a), (b) et (c) sont basés sur le poids total de chaque type de monomère utilisé par rapport au poids total de tous les monomères utilisés.

Le ou les copolymères (méth)acryliques (i) peuvent être présents en une quantité allant de 0,01 à 40% en poids, de préférence allant de 0,1 à 15 % en poids, et plus préférentiellement allant de 0,1 à 2% en poids, du poids total de la composition pour donner un effet repositionnable.

Le ou les copolymères (méth)acryliques (i) peuvent être sous forme d'émulsion ou de dispersion. Toutes les émulsions comprennent une phase continue et au moins une phase dispersée.

Par « dispersion », on entend généralement un système multiphasique où au moins une phase contient des particules discrètes distribuées dans une phase externe liquide, solide ou gazeuse. Une partie du polymère peut exister sous forme de particules discrètes dans une phase aqueuse. Les dispersions sont possibles grâce à l'utilisation de certains composants qui sont insolubles dans le système aqueux. Dans une dispersion, ce n'est pas nécessairement l'ensemble du polymère qui doit être insoluble dans l'eau ; une partie du polymère peut être soluble dans le mélange aqueux. Il peut être souhaitable qu'une dispersion reste stable dans des conditions ambiantes.

Dans un mode de mise en oeuvre, les dispersions sont stables à température ambiante pendant plus d'environ 30 jours, par exemple pendant plus d'environ 90 jours, de préférence pendant plus d'environ 180 jours, et mieux encore pendant plus d'environ 360 jours. Une dispersion est jugée stable dès lors que les particules discrètes de la phase interne restent distribuées dans l'ensemble de la substance de masse (phase externe).

Dans un mode de mise en oeuvre, de telles dispersions peuvent être mélangées avec d'autres dispersions ou d'autres additifs connus tels que des plastifiants, des pigments (tels que le noir de carbone), des sols de silice et d'autres agents de nivellement, agents mouillants, agents anti-mousse, et stabilisants connus.

Le ou les copolymères (méth)acryliques (i) utilisés dans la composition selon l'invention peuvent être préparés par des techniques de polymérisation conventionnelles, telles que la polymérisation en émulsion, comme cela est bien connu dans le domaine des polymères.

La polymérisation par l'intermédiaire des techniques d'émulsion peut nécessiter la présence d'un ou plusieurs émulsifiants afin de faciliter l'émulsification du mélange de monomères (que l'on peut également appeler agents émulsifiants ou tensioactifs). Les émulsifiants utiles pour la présente invention incluent ceux choisis parmi les tensioactifs anioniques, les tensioactifs non ioniques, et des mélanges de ceux-ci.

Les tensioactifs anioniques utiles comme émulsifiants incluent, mais sans s'y limiter, ceux dont la structure moléculaire inclut au moins une moitié hydrophobe choisie parmi les alkyles en C₆ à C₁₂, les alkylaryles en C₆ à C₁₂, et les alcényles en C₆ à C₁₂ et au moins un groupement anionique choisi parmi les sulfates, les sulfonates, les phosphates, les polyoxyéthylènesulfates, les polyoxyéthylènesulfonates, les polyoxyéthylènephosphates, etc., et les sels de tels groupements. Dans un mode de mise en oeuvre, lesdits sels sont choisis parmi les sels de métaux alcalins, les sels d'ammonium, les sels d'amino tertiaire, etc. Des exemples commerciaux représentatifs des tensioactifs anioniques utiles comme émulsifiants incluent les laurylsulfates de sodium, disponibles auprès de Stepan Chemical Co. sous forme de POLYSTEP B-3 ; les lauryléthersulfates de sodium, disponibles auprès de Stepan Chemical Co. sous forme de POLYSTEP B-12 ; les dodécylbenzènesulfonates de sodium, disponibles auprès de Rhodia Chimie sous forme de SIPONATE DS-10 ; et les alkylènepolyalcoxyammonium sulfates, disponibles auprès de PPG Industries sous forme de MAZON SAM-211.

Les tensioactifs non ioniques utiles comme émulsifiants incluent, mais sans s'y limiter, ceux dont la structure moléculaire comprend un produit de condensation d'une moitié aliphatique organique et/ou alkyle aromatique avec un oxyde d'alkylène hydrophile tel que l'oxyde d'éthylène. Le coefficient HLB (balance lipophile-hydrophile) de tensioactifs non ioniques utiles comme émulsifiants est d'environ 10 ou supérieur, par exemple d'environ 10 à environ 20. Le coefficient HLB d'un tensioactif est une expression de la balance de la taille et de la résistance des groupements hydrophiles (hydro-attracteurs ou polaires) et des groupements lipophiles (oléo-attracteurs ou non polaires) du tensioactif. Des exemples commerciaux des tensioactifs non ioniques utiles dans la présente invention incluent, mais sans s'y limiter, les nonylphénoxy ou octylphénoxy poly(éthylèneoxy)éthanols disponibles auprès de Rhodia Chimie sous forme respectivement de la série IGEPAL CA ou CO ; les éthoxylates d'alcools secondaires en C₁₁-C₁₅ disponibles auprès d'Union Carbide sous forme de la série TERGITOL 15-S ; et les esters d'acides gras du sorbitane oxyéthylénés disponibles auprès d'ICI Chemicals sous forme de la série de tensioactifs TWEEN.

Dans un mode de mise en oeuvre, une polymérisation en émulsion selon l'invention est réalisée en présence d'un ou plusieurs tensioactifs anioniques comme émulsifiants. Une gamme utile de concentration d'émulsifiant est de 0,5 à 8 % en poids, par exemple de 1 à 5 % en poids, sur la base du poids total de tous les monomères.

Selon un mode de réalisation de l'invention, les copolymères (méth)acryliques (i) sont des émulsions ou des dispersions de copolymères (méth)acryliques.

Les émulsions et les dispersions de copolymères (méth)acryliques (i) peuvent être préparées par un procédé semi-continu de polymérisation en émulsion. Dans le procédé, une fiole est chargée d'un mélange de monomères amorce comprenant de l'eau désionisée (DI), du tensioactif, des monomères de (méth)acrylate de butyle (a), des monomères de (méth)acrylate d'hydroxyalkyle (b), et les composants éventuels tels que les monomères copolymérisables (c), des agents de réticulation polyfonctionnels, des agents de transfert de chaîne, des modificateurs de pH et d'autres additifs. Le mélange est agité et chauffé sous une atmosphère inerte telle qu'une couverture d'azote. Lorsque le mélange a atteint la température d'induction, typiquement d'environ 50°C à environ 70°C, le premier amorceur est ajouté pour amorcer la polymérisation et on laisse la réaction se dérouler de façon exothermique. Lorsque la réaction d'amorçage est terminée, la température du lot (batch) est ensuite augmentée jusqu'à la température de réaction d'alimentation, d'environ 70°C à environ 85°C. A la température de réaction d'alimentation, la pré-émulsion de monomères comprenant de l'eau DI, du tensioactif, des monomères de (méth)acrylate de butyle (a), des monomères de (méth)acrylate d'hydroxyalkyle (b), et des composants éventuels tels que les monomères copolymérisables (c), des agents de réticulation polyfonctionnels, des agents de transfert de chaîne, et d'autres additifs est ajoutée à la fiole agitée sur une période de temps, typiquement de 2 à 4 heures, pendant que la température est maintenue. A la fin de la réaction d'alimentation, la deuxième charge d'amorceur, si on l'utilise, est ajoutée à la réaction pour réduire encore les monomères résiduels dans l'émulsion/la dispersion. Après une heure supplémentaire de chauffage, le mélange est refroidi jusqu'à température ambiante (environ 23°C) et l'émulsion/la dispersion est récupérée pour une évaluation.

Dans un mode de mise en oeuvre, le pH de l'émulsion/la dispersion est d'environ 2 à environ 3. L'acidité de l'émulsion/la dispersion peut être modifiée suivant la formation de l'émulsion/la dispersion à l'aide d'un ou plusieurs modificateurs de pH tels que des solutions basiques (par ex. des solutions d'hydroxyde de sodium, d'hydroxyde d'ammonium, etc.) ou des solutions tampons (par ex. du bicarbonate de sodium, etc.) à des taux d'acidité inférieurs. Dans un autre mode de mise en oeuvre, le pH est de 7 ou inférieur. Dans encore un autre mode de mise en oeuvre, le pH est dans la gamme de 2 à 6.

Dans un mode de mise en oeuvre de l'invention, les copolymères (méth)acryliques (i) peuvent être neutralisés dans l'émulsion/la dispersion et/ou dans la composition par un ou plusieurs agents neutralisants. Les agents neutralisants convenables peuvent être choisis parmi des bases organiques, minérales ou organo-minérales, telles que les aminométhylpropanols, les hydroxydes de sodium et de potassium, les amines primaires, secondaires et tertiaires, les ammoniacs, les dérivés de ceux-ci, et les associations de ceux-ci.

Selon un mode de réalisation, les émulsions (méth)acryliques de l'invention peuvent également contenir un ou plusieurs additifs classiques, tels que des plastifiants, des colorants, des anti-oxydants, et des stabilisants des U.V. De tels additifs peuvent être utilisés s'ils n'altèrent pas les propriétés repositionnables de la composition.

Selon un mode de mise en oeuvre de l'invention, le copolymère (méth)acrylique (i) a une température de transition vitreuse (Tg) allant d'environ - 100°C à environ 15°C. Selon la présente invention, la Tg du copolymère (méth)acrylique est obtenue à la suite de l'application du copolymère (méth)acrylique (i) dans un milieu aqueux ou hydro-alcoolique sur une matrice, puis du séchage jusqu'à poids constant. La température de transition vitreuse est déterminée par la méthode d'analyse thermique différentielle (DSC).

Dans un mode de mise en oeuvre de l'invention, le copolymère (méth)acrylique (i) est choisi parmi les copolymères (méth)acryliques non ioniques et faiblement anioniques. Faiblement anionique signifie que le taux de motifs issus de monomères anioniques dans le copolymère est inférieur à 5 % en poids.

Par huile, on entend un corps non aqueux liquide à température ambiante (22°C +/- 3°C) et pression atmosphérique (760 mm de Hg).

La composition conforme à l'invention comprend une ou plusieurs huiles non-siliconées qui peuvent être en particulier choisies parmi les huiles hydrocarbonées et/ou fluorées ou leurs mélanges.

On entend par "huile hydrocarbonée", une huile contenant principalement des atomes d'hydrogène et de carbone et éventuellement des atomes d'oxygène, d'azote, de soufre, de phosphore.

Comme huile hydrocarbonée, on peut notamment citer :
- les huiles hydrocarbonées d'origine végétale telles que les triglycérides constitués d'esters d'acides gras et de glycérol dont les acides gras peuvent avoir des longueurs de chaînes variées de C₄ à C₂₄, ces derniers pouvant être linéaires ou ramifiés, saturés ou insaturés ; ces huiles sont notamment les huiles de germe de blé, de tournesol, de pépins de raisin, de sésame, de maïs, d'abricot, de ricin, de karité, d'avocat, d'olive, de soja, l'huile d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ; ou encore les triglycérides des acides caprylique/caprique comme ceux vendus par la société Stéarineries Dubois ou ceux vendus sous les dénominations de Miglyol 810®, 812® et 818® par la société Dynamit Nobel,
- les éthers de synthèse ayant de 10 à 40 atomes de carbone ;
- les huiles hydrocarbonées sans hétéroatome, linéaires ou ramifiées, d'origine minérale ou synthétique telles que l'huile de paraffine, les polydécènes, le polyisobutène hydrogéné tel que le parléam, le squalane, et leurs mélanges;
- les esters liquides de synthèse comme les composés de formule R₁COOR₂ dans laquelle R₁ représente un radical linéaire ou ramifié comportant de 1 à 40 atomes de carbone et R₂ représente une chaîne hydrocarbonée notamment ramifiée contenant de 1 à 40 atomes de carbone à condition que la somme des atomes de carbone de R₁ et R₂ soit supérieure ou égale à 10, comme par exemple l'huile de Purcellin (octanoate de cétostéaryle), le myristate d'isopropyle, le palmitate d'isopropyle, le benzoate d'alcool en C₁₂ à C₁₅, le laurate d'hexyle, l'adipate de diisopropyle, l'isononanoate d'isononyle, le palmitate de 2-éthyl-hexyle, l'isostéarate d'isostéarate, des octanoates, décanoates ou ricinoléates d'alcools ou de polyalcools comme le dioctanoate de propylène glycol ; les esters hydroxylés comme le lactate d'isostéaryle, le malate de di-isostéaryle ; et les esters du pentaérythritol;
- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, le 2-undécylpentadécanol ;
- les acides gras supérieurs tels que l'acide oléique, l'acide linoléique, l'acide linolénique ;
et leurs mélanges.

Les huiles fluorées utilisables dans les compositions conformes à l'invention sont notamment des polyéthers fluorés.

De préférence, l'huile non-siliconée utilisée selon l'invention est choisie parmi une huile hydrocarbonée d'origine végétale et les esters liquides de synthèse, de préférence le myristate d'isopropyle.

La ou les huiles non-siliconées sont présentes dans la composition dans une teneur allant de 0,1 à 5 %, de préférence de 0,5 à 2 % en poids par rapport au poids total de la composition.

La composition fournit un effet repositionnable et est de préférence une composition rincée, par exemple, un après-shampooing
ou un shampooing, ce dernier pouvant ou non présenter un effet conditionneur.

Selon un premier mode de réalisation préféré de l'invention, la composition de l'invention comprend dans un véhicule cosmétiquement acceptable, (1) une ou plusieurs huiles non-siliconées et (2) un ou plusieurs copolymères (méth)acryliques comprenant : (a) des motifs issus d'un ou plusieurs monomères choisis parmi les monomères de (méth)acrylate de butyle, (b) des motifs issus d'un ou plusieurs monomères choisis parmi les monomères de (méth)acrylate d'hydroxyalkyle et éventuellement (c) des motifs issus d'un ou plusieurs monomères copolymérisables autres que lesdits monomères (a) et (b), et (3) un ou plusieurs tensioactifs.

Selon un second mode de réalisation de l'invention, la composition de l'invention comprend dans un véhicule cosmétiquement acceptable, (1) une ou plusieurs huiles non-siliconées et (2) un ou plusieurs copolymères (méth)acryliques comprenant : (a) des motifs issus d'un ou plusieurs monomères choisis parmi les monomères de (méth)acrylate de butyle, (b) des motifs issus d'un ou plusieurs monomères choisis parmi les monomères de (méth)acrylate d'hydroxyalkyle et éventuellement (c) des motifs issus d'un ou plusieurs monomères copolymérisables autres que lesdits monomères (a) et (b), et (3) un ou plusieurs agents conditionneurs différents des huiles non-siliconées (1).

La composition comprenant une ou plusieurs huiles non-siliconées et un ou plusieurs copolymères (méth)acryliques, tels que décrits ci-dessus, peut se présenter sous forme de sprays, d'aérosols, de mousses, de gels, de lotions, de crèmes, de dispersions ou d'émulsions.

La composition rincée peut se présenter sous toutes les formes classiques de compositions cosmétiques rincées y compris, mais sans s'y limiter, les shampooings, après-shampooings, lotions de rinçage des cheveux, compositions de permanente, compositions de teinture des cheveux, produits à utiliser avant ou après un traitement de teinture des cheveux, produits à utiliser avant ou après un traitement de permanente, compositions de défrisage, produits à utiliser avant ou après un traitement de défrisage, et des associations de ceux-ci. Un shampooing présente un effet nettoyant sur les cheveux et peut également présenter un effet conditionneur. Un après-shampooing présente un effet conditionneur sur les cheveux sans effet nettoyant notable.

Selon un mode de mise en oeuvre de l'invention, le copolymère (méth)acrylique peut être présent en une quantité allant de 0,01 à 40, de préférence de 0,1 à 15 % en poids, et plus préférentiellement de 0,1 à 2 % en poids, du poids total de la composition pour donner un effet repositionnable.

La composition peut en outre comprendre tout véhicule cosmétiquement acceptable qui ne gêne pas substantiellement les propriétés adhésives de la composition selon l'invention. Le choix de véhicule est adapté au procédé d'application choisi. Dans un mode de mise en oeuvre, le véhicule cosmétiquement acceptable peut être choisi parmi l'eau, les solvants miscibles à l'eau tels que les alcools inférieurs, par ex. les alcools aliphatiques à chaîne ramifiée et droite en C₁ à C₄, et des associations de ceux-ci. Dans un mode de mise en oeuvre, le copolymère (méth)acrylique est insoluble dans le véhicule cosmétiquement acceptable.

Le véhicule peut également comprendre un ou plusieurs solvants supplémentaires. Par exemple, on peut utiliser d'autres solvants s'évaporant rapidement, tels que l'hexaméthyldisiloxane (HMDS) ; les silicones cycliques (D4 et D5) ; les alcanes en C₄ - C₁₀ dont les isoparaffines telles que Permethyl 97A et Isopar C ; l'acétone ; les hydrofluoroéthers (HFE), etc.

La composition selon l'invention peut en outre comprendre un ou plusieurs constituants connus en cosmétique qui ne gênent pas substantiellement les propriétés repositionnables de la composition. De tels constituants peuvent être choisis parmi les, mais ne sont pas limités aux : agents réducteurs (tels que les thiols) ; agents oxydants ; les polymères anioniques ; silanes (tels que l'aminopropyltriéthoxysilane) ; matières grasses ; épaississants ; plastifiants ; agents hydratants ; charges ; filtres solaires (tels que filtres UV) ; agents actifs de soin des cheveux ; parfums ; conservateurs ; polyols ; protéines ; provitamines ; vitamines ; colorants ; et agents de modification du pH.

Selon le second mode de réalisation de l'invention, la composition peut également comprendre un ou plusieurs agents conditionneurs. On entend par « agent conditionneur » tel qu'utilisé ici tout agent dont la fonction est d'améliorer les propriétés cosmétiques des cheveux, par exemple la douceur, la facilité de démêlage, le toucher, et l'absence d'électricité statique. Dans une variante, l'agent conditionneur est choisi parmi les polymères non-siliconés cationiques, non-ioniques, amphotères, et les silicones.

Selon un mode de mise en oeuvre particulier, l'agent conditionneur est choisi parmi les polymères non-siliconés tels que les polymères cationiques, amphotères (tels que zwitterioniques), et non ioniques, de préférence les polymères cationiques et des associations de ceux-ci. On entend par « polymère » tel qu'utilisé ici les homopolymères et les copolymères, les copolymères étant issus de plus d'un type de monomère, par exemple de deux, trois, quatre types de monomères différents ou plus.

Les polymères cationiques comprennent des groupements cationiques ou des groupements pouvant être transformés en groupements cationiques. Des exemples convenables de polymères cationiques, qui peuvent être utilisés selon la présente invention, sont ceux pouvant être choisis parmi les polymères comprenant au moins un groupement choisi parmi les groupements amines primaires, les groupements amines secondaires, les groupements amines tertiaires, et les groupements amines quaternaires, dans lesquels ledit au moins un groupement fait partie de la chaîne polymère ou est directement relié à celle-ci, ayant un poids moléculaire moyen en poids allant d'environ 500 à environ 5 000 000, par exemple d'environ 1 000 à environ 3 000 000.

Parmi ces polymères, on peut citer plus particulièrement les polymères conditionneurs cationiques suivants :
(1) les homopolymères et copolymères issus de monomères choisis parmi les esters (méth)acryliques et les amides (méth)acryliques comprenant des motifs d'au moins une des formules suivantes : 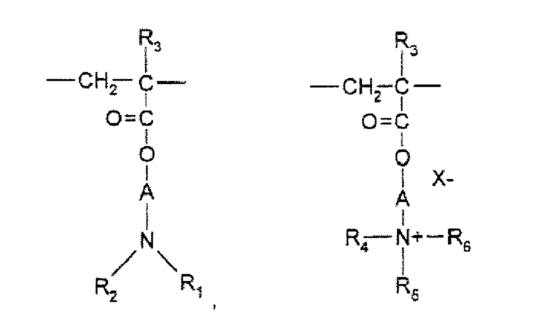 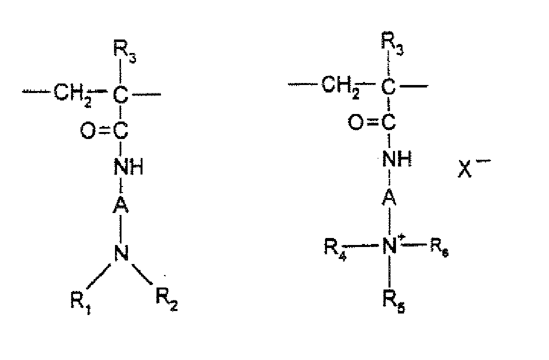 dans lesquelles chaque R3 est choisi indépendamment parmi l'hydrogène et des groupements CH₃ ; chaque A est choisi indépendamment parmi des groupements alkyle linéaires et ramifiés comprenant de 1 à 6 atomes de carbone et des groupements hydroxyalkyle comprenant de 1 à 4 atomes de carbone ; chaque R₄, R₅ et R₆ est choisi indépendamment parmi les groupements alkyle comprenant de 1 à 18 atomes de carbone et les groupements benzyle ; chaque R₁ et R₂ est choisi indépendamment parmi l'hydrogène et les groupements alkyle comprenant de 1 à 6 atomes de carbone ; et que chaque X- est choisi indépendamment parmi les anions de sulfate de méthyle et les anions d'halogénure, tels que les anions chlorure ou bromure.
   Dans un mode de mise en oeuvre, les copolymères de la famille (1) comprennent en outre au moins un motif issu de monomères choisis parmi les (méth)acrylamides, les diacétones (méth)acrylamides, les (méth)acrylamides substitués sur l'azote par un groupement choisi parmi les alkyle inférieurs, les acides (méth)acryliques, les esters d'acides (méth)acryliques, les vinyllactames tels que la vinylpyrrolidone et le vinylcaprolactame, et les esters vinyliques.
   Ainsi, parmi ces copolymères conditionneurs cationiques de la famille (1), on peut citer :
   - les copolymères d'acrylamide et de méthacrylate de diméthylaminoéthyle quaternisé avec du sulfate de diméthyle ou avec un halogénure de diméthyle, tels que Hercofloc disponible auprès de la société Hercules ;
   - les copolymères d'acrylamide et de chlorure de méthacryloyloxyéthyltriméthylammonium qui sont divulgués, par exemple, dans le document EP-A-080 976 et vendus par exemple sous le nom Bina Quat P 100 par la société Ciba-Geigy ;
   - les copolymères d'acrylamide et de méthosulfate de méthacryloyloxyéthyltriméthylammonium, tels que Reten disponible auprès de la société Hercules ;
   - éventuellement les copolymères vinylpyrrolidone/(méth)acrylate de dialkyl-aminoalkyle, qui sont divulgués, par exemple, dans les brevets français 2 077 143 et 2 393 573 et vendus, par exemple, sous le nom « Gafquat » par la société ISP, tels que, par exemple, « Gafquat 734 » ou « Gafquat 755 », ou bien les produits dénommés « Copolymère 845, 958 et 937 » ;
   - les terpolymères méthacrylate de diméthylaminoéthyle/vinylcaprolactame/vinylpyrrolidone tels que le produit vendu sous le nom Gaffix VC 713 par la société ISP ;
   - les copolymères vinylpyrrolidone/méthacrylamidopropyldiméthylamine vendus en particulier sous le nom Styleze CC 10 par ISP, et
   - le copolymère vinylpyrrolidone/diméthylamino-propylméthacrylamide quaternisé, tel que le produit vendu sous le nom « Gafquat HS 100 » par la société ISP.
(2) les dérivés d'éther de cellulose contenant des groupements ammonium quaternaires décrits dans le brevet français 1 492 597. Des exemples incluent les polymères vendus sous les noms « JR » (JR 400, JR 125 et JR 30M) ou « LR » (LR 400 et LR 30M) par la société Amerchol. Ces polymères sont également définis dans le dictionnaire CTFA comme des ammoniums quaternaires d'hydroxyéthylcellulose ayant réagi avec un époxyde substitué par un groupement triméthylammonium.
(3) les dérivés de cellulose cationiques tels que les copolymères de cellulose et les dérivés de cellulose greffés avec un monomère hydrosoluble d'ammonium quaternaire, et divulgués notamment dans le brevet U.S. 4 131 576. Des exemples incluent des hydroxyalkylcelluloses, par exemple les hydroxyméthyl-, hydroxyéthyl-, et hydroxypropyl-celluloses greffées notamment avec un sel de méthacryloyléthyltriméthylammonium, de méthacrylamidopropyltriméthylammonium, ou de diallyldiméthylammonium.
   Les produits commercialisés répondant à cette définition sont plus particulièrement les produits vendus sous le nom « Celquat L 200 » et « Celquat H 100 » par la société National Starch.
(4) les chitosanes ou leurs sels. Les sels pouvant être utilisés sont notamment l'acétate, le lactase, le glutamate, le gluconate ou le pyrrolidone-carboxylate de chitosane.
   Parmi ces composés, on peut citer le chitosane ayant un degré de déacétylation de 90,5 % en poids, vendu sous le nom Kytan Crude Standard par la société Aber Technologies, et le pyrrolidone-carboxylate de chitosane vendu sous le nom Kytamer PC par la société Amerchol.
(5) les polysaccharides quaternisés, divulgués plus particulièrement dans les brevets U.S. n° 3 589 578 et 4 031 307, tels que les gommes de guar comprenant des groupements cationiques trialkylammonium. Des exemples incluent des gommes de guar modifiées par un sel (p. ex., le chlorure) de 2,3-époxypropyltriméthylammonium.
   De tels produits sont vendus notamment sous les noms commerciaux Jaguar C13 S, Jaguar C 15, Jaguar C 17, et Jaguar C162 par la société Rhodia Chimie.
(6) les copolymères comprenant des motifs pipérazinyle des groupements divalents alkylène ou hydroxyalkylène à chaînes droite et ramifiée, éventuellement interrompus par un ou plusieurs atomes d'oxygène, atomes de soufre, atomes d'azote, ou par des cycles aromatiques, et des cycles hétérocycliques. Des exemples incluent des produits d'oxydation et de quaternisation de ces copolymères. De tels polymères sont décrits notamment dans les brevets français 2 162 025 et 2 280 361.
(7) les polyaminoamides hydrosolubles, qui peuvent être préparés par polycondensation d'un composé acide avec une polyamine. Ces polyaminoamides peuvent être réticulés par au moins un composé choisi parmi les épihalohydrines, les diépoxydes, les dianhydrides, les dianhydrides insaturés, les dérivés bis-insaturés, les bis-halohydrines, les bis-azétidiniums, les bis-haloacyldiamines, les bis-halogénures d'alkyle, et les oligomères résultant de la réaction d'un composé difonctionnel, réactif vis-à-vis d'un composé choisi parmi les bis-halohydrines, les bis-azétidiniums, les bis-haloacyldiamines, les bis-halogénures d'alkyle, les épihalohydrines, les diépoxydes, et les dérivés bis-insaturés. L'agent réticulant peut être utilisé dans des proportions allant d'environ 0,025 à environ 0,35 mole par groupement amine du polyaminoamide. Dans un mode de mise en oeuvre, ces polyaminoamides sont alkylés et/ou, s'ils contiennent une ou plusieurs fonctions amines tertiaires, quaternisés. De tels polymères sont décrits notamment dans les brevets français 2 252 840 et 2 368 508.
(8) les dérivés de polyaminoamides résultant de la condensation de polyalkylènes-polyamines avec des acides polycarboxyliques, suivie d'une alkylation par des agents difonctionnels. Des exemples incluent des polymères acide adipique/dialkylaminohydroxyalkyldialkylènetriamine dans lesquels les groupements alkyle sont des groupements alkyle sont des groupements alkyle en C₁-C₄ (tels que méthyle, éthyle, et propyle), tels que les polymères acide adipique/diméthylaminohydroxypropyldiéthylènetriamine vendus sous le nom « Cartaretine F, F4 et F8 » par la société Sandoz. De tels polymères sont décrits notamment dans le brevet français 1 583 363.
(9) les polymères obtenus par réaction d'une polyalkylène-polyamine contenant deux groupements amines primaires et au moins un groupement amine secondaire, avec un acide dicarboxylique choisi parmi les acides diglycoliques et les acides dicarboxyliques aliphatiques saturés ayant de 3 à 8 atomes de carbone. Le rapport molaire entre la polyalkylène-polyamine et l'acide dicarboxylique est compris entre environ 0,8:1 et environ 1,4:1. Le polyaminoamide en résultant est mis en réaction avec l'épichlorohydrine dans un rapport molaire d'épichlorohydrine par rapport au groupement amine secondaire du polymaminoamide compris entre environ 0,5:1 et environ 1,8:1. De tels polymères sont décrits notamment dans les brevets U.S. n° 3 227 615 et 2 961 347.
   Des polymères de ce type sont vendus notamment sous le nom « Hercosett 57 » par la société Hercules Inc., ou bien sous le nom « PD 170 » ou « Delsette 101 » par la société Hercules dans le cas du copolymère acide adipique/époxypropyl/diéthylènetriamine.
(10) les cyclopolymères d'alkyldiallylamine et/ou de dialkyldiallylammonium, tels que les homopolymères ou copolymères contenant, comme constituant principal de la chaîne, des motifs choisis parmi les formules (I) et/ou (II) : 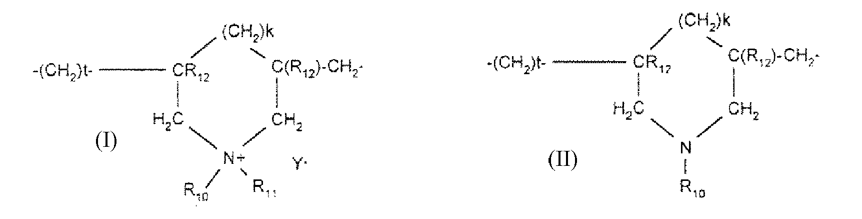 dans lesquelles k et t sont soit 0 soit 1 et la somme k + t est égale à 1 ; chaque R₁₂ est choisi indépendamment parmi l'hydrogène et les groupements méthyle ; R₁₀ et R₁₁ sont choisis indépendamment parmi les groupements alkyle ayant de 1 à 8 atomes de carbone (tels que de 1 à 4 atomes de carbone), les groupements hydroxyalkyle en C₁-C₅ et les groupements amidoalkyle en C₁-C₄, ou R₁₀ et R₁₁, pris ensemble avec l'atome d'azote auquel ils sont liés, peuvent former un groupement hétérocyclique tel que pipéridyle et morpholinyle ; Y⁻ est choisi indépendamment parmi les anions tels que les anions bromure, chlorure, acétate, borate, citrate, tartrate, bisulfate, bisulfite, sulfate, et phosphate. Ces polymères sont décrits notamment dans le brevet français 2 080 759 et dans son certificat d'addition 2 190 406.
   Parmi les polymères définis ci-dessus, on peut citer plus particulièrement l'homopolymère de chlorure de diméthyldiallylammonium vendu sous le nom « Merquat 100 » par la société Nalco (ses homologues de faible masse moléculaire moyenne en poids) et les copolymères de chlorure de diallyldiméthylammonium et d'acrylamide, vendus sous le nom « Merquat 550 ».
(11) les polymères de diammonium quaternaire contenant des motifs récurrents répondant à la formule (III) : 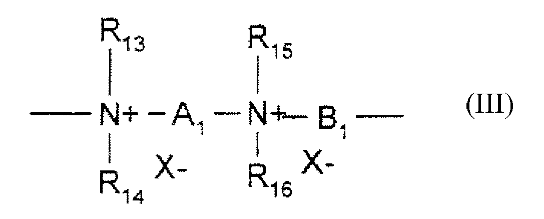 dans laquelle R₁₃, R₁₄, R₁₅ et R₁₆ sont choisis indépendamment parmi des groupements aliphatique en C₁-C₂₀, alicyclique en C₁-C₂₀ ou arylaliphatique en C₁-C₂₀, et des groupements hydroxyalkyle aliphatiques inférieurs ; ou bien R₁₃, R₁₄, R₁₅ et R₁₆, ensemble ou séparément, constituent avec les atomes d'azote auxquels ils sont liés des hétérocycles contenant éventuellement un second hétéroatome autre que l'azote ; ou bien R₁₃, R₁₄, R₁₅ et R₁₆ sont choisis indépendamment parmi des groupements alkyle en C₁-C₆ linéaires et ramifiés, substitués par au moins un groupement choisi parmi les groupements nitriles, esters, acyles, amides, -CO-O-R₁₇-D, et des groupements -CO-NH-R₁₇-D, où R₁₇ est un alkylène et D est un groupement ammonium quaternaire ; A₁ et B₁ sont choisis indépendamment parmi des groupements polyméthylène linéaires et ramifiés, saturés et insaturés, contenant de 2 à 20 atomes de carbone et pouvant contenir, liés à ou intercalés dans la chaîne principale, au moins un groupement choisi parmi les cycles aromatiques, l'oxygène, le soufre, les sulfoxydes, les sulfones, les disulfures, les amino, les alkylamino, les hydroxy, les ammonium quaternaires, les uréido, les amides et les esters ; X⁻ est choisi indépendamment parmi les anions issus des acides minéraux ou organiques. Dans un mode de mise en oeuvre, X⁻ est un anion tel que le chlorure et le bromure.
   A₁, R₁₃, et R₁₅ peuvent former, avec les deux atomes d'azote auxquels ils sont liés, un cycle pipérazinique.
   De manière alternative, A₁ est choisi parmi les groupements alkylène ou hydroxyalkylène linéaires et ramifiés, saturés et insaturés, et B₁ est choisi parmi des groupements (CH₂)ₙ-CO-E-OC-(CH₂)ₙ- dans lesquels n varie de 1 à 6 et E est choisi parmi :
   a) des restes de glycol de formule : -O-Z-O-, où Z est choisi parmi des groupements hydrocarbonés linéaires et ramifiés et des groupements répondant à l'une des formules suivantes :

      -(CH₂-CH₂-O)ₓ-CH₂-CH₂-

      et

      -[CH₂-CH(CH₃)-O]_{y}-CH₂-CH(CH₃)-

      où x et y sont des entiers allant indépendamment de 1 à 4 et représentant un degré de polymérisation défini et unique ou un nombre quelconque de 1 à 4 et représentant un degré de polymérisation moyen ;
   b) des restes diamines bis-secondaires, tels qu'un dérivé de pipérazine ;
   c) des restes de diamines bis-primaires, de formule : -NH-Y-NH-, où Y est choisi parmi des groupements hydrocarbonés linéaires et ramifiés et CH₂-CH₂-S-S-CH₂-CH₂- ; et
   d) des groupements uréylène de formule : -NH-CO-NH-.
   Ces polymères ont généralement une masse moléculaire moyenne en nombre allant d'environ 1000 à environ 100 000. Des exemples de ces polymères de ce type sont décrits dans les brevets U.S. n° 2 273 780, 2 375 853, 2 388 614, 2 454 547, 3 206 462, 2 261 002, 2 271 378, 3 874 870, 4 001 432, 3 929 990, 3 966 904, 4 005 193, 4 025 617, 4 025 627, 4 025 653, 4 026 945, et 4 027 020.
   Dans un mode de mise en oeuvre, les polymères conditionneurs cationiques de la famille (11) sont constitués de motifs récurrents répondant à la formule : 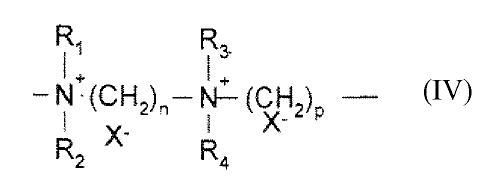 dans laquelle R₁, R₂, R₃, et R₄ sont choisis indépendamment parmi le radical alkyle en C₁-C₄ et hydroxyalkyle en C₁-C₄, n et p sont des entiers allant indépendamment d'environ 2 à environ 20 et X⁻ est un anion choisi parmi les acides minéraux et organiques.
   Dans un mode de mise en oeuvre, R₁, R₂, R₃, et R₄ sont choisis parmi des groupements méthyle ; n = 3 ; p = 6 ; et X = Cl, dénommé chlorure d'hexadiméthrine selon la nomenclature de l'INCI (CTFA).
(12) les polymères de polyammonium quaternaires constitués de motifs de formule (V) : 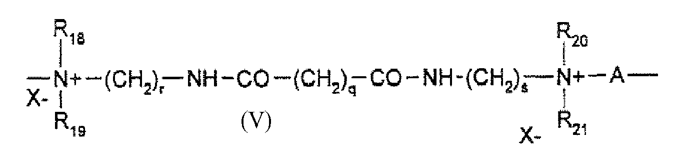 dans laquelle R₁₈, R₁₉, R₂₀, et R₂₁ sont choisis indépendamment parmi l'hydrogène, les groupements méthyle, éthyle, propyle, β-hydroxyéthyle, β-hydroxypropyle, et -CH₂CH₂(OCH₂CH₂)ₚOH, où p est un entier allant de 0 à 6 sous réserve que R₁₈, R₁₉, R₂₀, et R₂₁ ne sont pas simultanément l'hydrogène ; r et s sont des entiers allant indépendamment de 1 à 6 ; q est un entier allant de 0 à 34 ; X⁻ est choisi parmi les anions, tels que les halogénures ; A est choisi parmi des groupements dihalogénures tels que - CH₂-CH₂-O-CH₂-CH₂-.
   De tels composés sont notamment décrits dans la demande de brevet EP-A-122 324. Parmi ces produits, on peut citer par exemple « Mirapol® A 15 », « Mirapol® AD1 », « Mirapol® AZ1 » et « Mirapol® 175 » vendus par la société Miranol.
(13) les polymères quaternaires de vinylpyrrolidone et de vinylimidazole tels que, par exemple, les produits vendus sous les noms Luviquat® TFC, FC 905, FC 550 et FC 370 par la société BASF.
(14) les polyamines telles que Polyquart® H vendu par Henkel référencées sous le nom de « Polyethylene glycol (15) tallow polyamine » dans le dictionnaire CTFA.
(15) les polymères réticulés ou non réticulés de sels de méthacryloyloxyalkyl(C₁-C₄)trialkyl(C₁-C₄)ammonium tels que les polymères obtenus par homopolymérisation du méthacrylate de diméthylaminoéthyle quaternisé par le chlorure de méthyle ou par copolymérisation de l'acrylamide avec le méthacrylate de diméthylaminoéthyle quaternisé par un halogénure (tel que chlorure) de méthyle, l'homopolymérisation ou la copolymérisation étant suivie d'une réticulation par un composé à insaturation oléfinique, notamment le méthylènebisacrylamide. Dans un mode de mise en oeuvre, on utilise un copolymère réticulé acrylamide/chlorure de méthacryloyloxyéthyl-triméthylammonium (20/80 en poids) sous forme d'une dispersion contenant 50 % en poids dudit copolymère dans de l'huile minérale. Cette dispersion est vendue sous le nom « Salcare® SC 92 » par la société Allied Colloids. Dans un autre mode de mise en oeuvre, on peut également utiliser un homopolymère réticulé du chlorure de méthacryloyloxyéthyl-triméthylammonium contenant environ 50 % en poids de l'homopolymère dans de l'huile minérale ou dans un ester liquide. Ces dispersions sont vendues sous les noms « Salcare® SC 95 » et « Salcare® SC 96 » par la société Allied Colloids.

D'autres polymères conditionneurs cationiques utilisables dans le cadre de l'invention sont des protéines cationiques ou des hydrolysats de protéines cationiques, des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Dans un mode de mise en oeuvre, les polymères conditionneurs cationiques sont choisis parmi les dérivés d'éther de cellulose quaternaires tels que les produits vendus sous le nom « JR 400 » par la société Amerchol ; la gomme de guar quaternisée telle que les produits vendus sous le nom « Jaguar C 13 S » par la société Rhodia ; les cyclopolymères cationiques, notamment les homopolymères ou les copolymères du chlorure de diméthyldiallylammonium, vendus sous les noms « Merquat 100 », « Merquat 550 » et « Merquat S » par la société Nalco, les polymères quaternaires de vinylpyrrolidone et de vinylimidazole ; les polymères non réticulés et réticulés de sels de méthacryloyloxyalkyl(C₁-C₄)trialkyl(C₁-C₄)ammonium tels que les produits vendus sous le nom « Salcare SC 96 » par la société NALCO ; et des mélanges de ceux-ci. Selon un mode de réalisation particulièrement préféré, les compositions selon l'invention comprennent au moins un polymères cationique choisi parmi les homopolymères ou les copolymères du chlorure de diméthyldiallylammonium.

Selon l'invention, le(s) polymère(s) cationique(s) peut (peuvent) représenter de 0,001 % à 20 % en poids, par exemple de 0,01 % à 10 % en poids, encore par exemple de 0,1 % à 2 % en poids, par rapport au poids total de la composition.

Les polymères amphotères utilisables conformément à l'invention peuvent être choisis parmi les polymères comprenant des motifs X et Y, répartis statistiquement dans la chaîne polymère, où le motif X est choisi parmi les motifs issus d'au moins un monomère comprenant au moins une fonction basique, notamment un atome d'azote basique, et où le motif Y est choisi parmi les motifs issus d'au moins un monomère acide comprenant au moins un groupement choisi parmi les groupements carboxy et les groupements sulfo, ou bien où chaque motif X et Y est choisi indépendamment parmi les groupements issus de monomères zwitterioniques de carboxybétaïne et de sulfobétaïne. Dans un autre mode de mise en oeuvre, les polymères amphotères pouvant être utilisés conformément à l'invention peuvent être choisis parmi les polymères comprenant des motifs X et Y, chaque motif X et Y est choisi indépendamment parmi au moins une chaîne polymère cationique comprenant au moins un groupement choisi parmi des groupements amines primaires, des groupements amines secondaires, des groupements amines tertiaires, et des groupements amines quaternaires, dans laquelle au moins l'un des groupements amines comprend un groupement choisi parmi les groupements carboxy et sulfo reliés par l'intermédiaire d'un groupement hydrocarboné, ou bien les motifs X et Y, identiques ou différents, font partie d'une chaîne d'au moins un polymère comprenant un motif α,β-dicarboxy éthylène, où au moins l'un des groupements carboxy a été mis en réaction avec une polyamine comprenant au moins un groupement choisi parmi des groupements amines primaires et secondaires.

Dans un mode de mise en oeuvre, les polymères amphotères répondant à la définition donnée ci-dessus sont choisis parmi les polymères suivants :
(1) les polymères résultant de la copolymérisation d'un monomère issu d'un composé vinylique portant un groupement carboxy tel que les acides (méth)acryliques, les acides maléiques, et les acides α-chloracryliques et d'un monomère basique issu d'un composé vinylique substitué comprenant au moins un atome basique tel que le dialkylaminoalkyl-(méth)acrylate et le dialkylaminoalkyl-(méth)acrylamide. De tels composés sont divulgués dans le brevet U.S. n° 3 836 537.
(2) les polymères comprenant des motifs issus :
   a) d'au moins un monomère choisi parmi les (méth)acrylamides substitués sur l'azote par un groupement alkyle,
   b) d'au moins un comonomère acide comprenant au moins un groupement carboxy réactif et
   c) d'au moins un comonomère basique tel que des esters comprenant au moins un substituant choisi parmi des substituants amines primaires, secondaires, tertiaires et quaternaires des acides (méth)acryliques, et le produit de quaternisation du méthacrylate de diméthylaminoéthyle avec le sulfate de diméthyle ou diéthyle.
   Le monomère (méth)acrylamide N-substitué cité dans (a) est choisi plus particulièrement parmi les (méth)acrylamides N-substitués
   dans lesquels les groupements alkyle comprennent de 2 à 12 atomes de carbone, tels le N-éthylacrylamide, le N-tert-butylacrylamide, le N-tert-octylacrylamide, le N-octylacrylamide, le N-décylacrylamide, le N-dodécylacrylamide, et les méthacrylamides correspondants.
   Le comonomère acide cité dans (b) est choisi plus particulièrement parmi les acides (méth)acryliques, les acides crotoniques, les acides itaconiques, les acides maléiques, les acides fumariques, les monoesters d'alkyle en C₁-C₄ d'acide maléique, les monoesters d'alkyle en C₁-C₄ d'acide fumarique, les monoesters d'alkyle en C₁-C₄ d'anhydride maléique, et les monoesters d'alkyle en C₁-C₄ d'anhydride fumarique.
   Le comonomère basique cité dans (c) est choisi plus particulièrement parmi les méthacrylates d'aminoéthyle, de butylaminoéthyle, de N,N'-diméthylamino éthyle, et de N-tert-butylaminoéthyle.
   Dans un mode de mise en oeuvre, le polymère amphotère est choisi parmi les copolymères dont le nom CTFA (4e éd., 1991) est octylacrylamide/acrylates/butylaminoethyl methacrylate copolymer, tels que les produits vendus sous le nom Amphomer ou Lovocryl 47 par la société National Starch.
(3) les polyaminoamides réticulés et alkylés partiellement ou totalement issus de polyaminoamides de formule générale :

   ⁅ CO-R₁₀-CO-Z⁆ (VII)

   dans laquelle R₁₀ représente un groupement divalent issu d'un acide dicarboxylique saturé, d'un acide aliphatique mono- ou dicarboxylique comprenant une double liaison éthylénique, d'un ester d'un alcanol inférieur (ayant de 1 à 6 atomes de carbone) de ces acides, ou d'un groupement issu de l'addition de l'un quelconque desdits acides avec une amine bisprimaire ou bis-secondaire ; et Z désigne un groupement polyalkylène-polyamine bisprimaire, mono- ou bis-secondaire et représente par exemple :
   a) dans les proportions d'environ 60 % en mole à 100 % mole, le groupement : 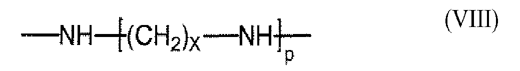 où x=2 et p=2 ou 3, ou bien x=3 et p=2 et où ce groupement est issu de diéthylènetriamine, de triéthylènetétraamine, ou de dipropylènetriamine ;
   b) dans les proportions de 0 % en mole à environ 40 % en mole, le groupement (VIII) ci-dessus, dans lequel x=2 et p=1 et qui dérive de l'éthylènediamine, ou le groupement issu de la pipérazine ; 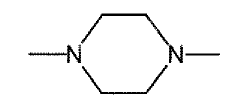
   c) dans les proportions de 0 % en mole à environ 20 % en mole, le groupement -NH-(CH₂)₆-NH- issu d'hexaméthylènediamine, ces polyaminoamines étant réticulées par addition d'un agent réticulant bifonctionnel choisi parmi les épihalohydrines, les diépoxydes, les dianhydrides et les dérivés bis-insaturés, à l'aide d'environ 0,025 mole à environ 0,35 mole d'agent réticulant par groupement amine du polyaminoamide et alkylées par l'action d'acide acrylique, d'acide chloroacétique ou d'une alcanesultone, ou leurs sels.
   Dans un mode de mise en oeuvre, les acides carboxyliques saturés sont choisis parmi les acides ayant de 6 à 10 atomes de carbone, tels que l'acide adipique, l'acide 2,2,4-triméthyladipique, l'acide 2,4,4-triméthyladipique, l'acide téréphtalique, et les acides à double liaison éthylénique comme par exemple l'acide acrylique, l'acide méthacrylique et l'acide itaconique.
   Dans un mode de mise en oeuvre, les alcanesultones utilisées dans l'alkylation sont choisies parmi la propanesultone et la butanesultone et les sels des agents d'alkylation sont choisis parmi les sels de sodium et de potassium.
(4) les polymères comprenant des motifs zwitterioniques de formule : 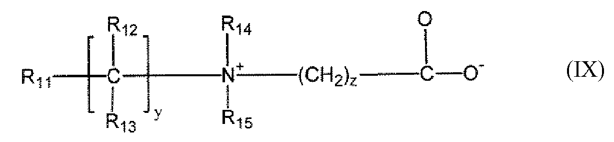 dans laquelle R₁₁ est choisi parmi les groupements insaturés polymérisables tels que des groupements (méth)acrylate et (méth)acrylamide ; y et z sont choisis indépendamment des entiers de 1 à 3 ; R₁₂ et R₁₃ sont choisis indépendamment parmi l'hydrogène, des groupements méthyle, des groupements éthyle et des groupements propyle ; R₁₄ et R₁₅ sont choisis indépendamment parmi l'hydrogène et les groupements alkyle, où la somme des atomes de carbone dans R₁₄ et R₁₅ est inférieure ou égale à 10.
   Les polymères comprenant de tels motifs peuvent également comprendre des motifs issus de monomères non zwitterioniques, tels que le (méth)acrylate de diméthylaminoéthyle, le (méth)acrylate de diéthylaminoéthyle, les (méth)acrylates d'alkyle, les (méth)acrylamides, et les acétates de vinyle.
   A titre d'exemple, on peut citer le copolymère méthacrylate de méthyle/diméthylcarboxyméthylammonio-éthyl-méthacrylate de méthyle tel que le produit vendu sous le nom Diaformer Z301 par la société Sandoz.
(5) les polymères issus du chitosane comprenant des motifs monomères répondant aux formules suivantes : 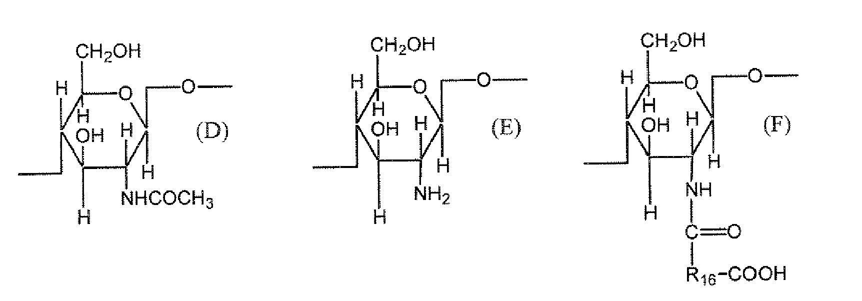 le motif D étant présent dans des proportions allant de 0 % à environ 30 %, le motif E dans des proportions allant d'environ 5 % à environ 50 % et le motif F dans des proportions allant d'environ 30 % à environ 90 %, étant entendu que dans ce motif F, R₁₆ représente un groupement de formule : 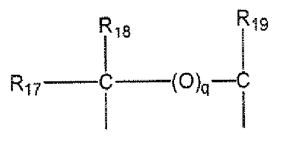 dans laquelle, si q=0, R₁₇, R₁₈ et R₁₉, identiques ou différents, sont choisis parmi l'hydrogène, des groupements méthyle, des groupements hydroxy, des groupements acétoxy, des résidus amino, des résidus monoalkylamine, et des résidus dialkylamine, éventuellement interrompus par un ou plusieurs atomes d'azote et/ou éventuellement substitués par un ou plusieurs groupements amine, hydroxy, carboxy, alkylthio, ou sulfo, et des résidus alkylthio dans lesquels le groupement alkyle porte un résidu amino, au moins l'un de R₁₇, R₁₈ et R₁₉ étant, dans ce cas, l'hydrogène ; ou si q=1, R₁₇, R₁₈ et R₁₉ représentent chacun l'hydrogène, et les sels formés par ces composés avec des bases ou des acides.
(6) Les polymères issus de la N-carboxyalkylation du chitosane, tels que le N-(carboxyméthyl)chitosane ou le N-(carboxybutyl)chitosane vendu sous le nom « Evalsan » par la société Jan Dekker.
(7) Les polymères répondant à la formule générale (X), par exemple divulgués dans le brevet français 1.400.366 : 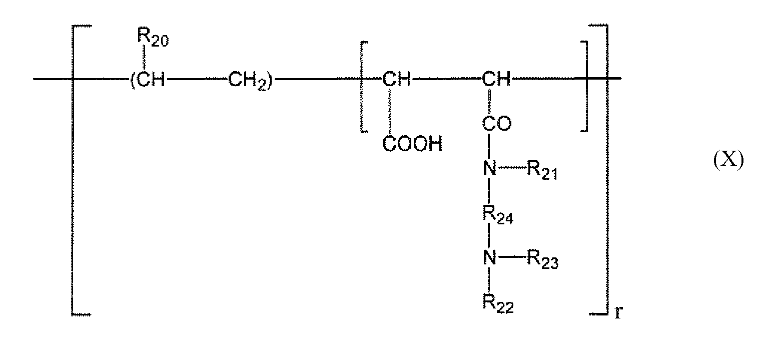 dans laquelle R₂₀ est choisi parmi l'hydrogène, les groupements CH₃O, CH₃CH₂O et phényle ; R₂₁ est choisi parmi l'hydrogène et les groupements alkyle inférieurs tels que méthyle ou éthyle ; R₂₂ est choisi parmi l'hydrogène et les groupements alkyle inférieurs tels que méthyl ou éthyle ; et R₂₃ est choisi parmi les groupements alkyle inférieurs tels que méthyle ou éthyle et les groupements répondant à la formule :-R₂₄-N(R₂₂)₂, où R₂₄ est choisi parmi les groupements -CH₂-CH₂-, -CH₂-CH₂-CH₂-, et -CH₂-CH(CH₃)- et R₂₂ a les mêmes significations que ci-dessus, et les homologues supérieurs de ces groupements comprenant jusqu'à 6 atomes de carbone.
(8) Les polymères amphotères du type -D-X-D-X- choisis parmi :
   a) les polymères obtenus par la réaction de l'acide chloroacétique ou le chloroacétate de sodium avec les composés comprenant au moins un motif de formule :

      -D-X-D-X-D- (XI)

      dans laquelle D désigne un groupement : 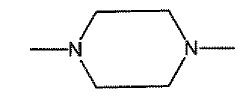 et X désigne le symbole E ou E'. E et E', identiques ou différents, désignent un groupement bivalent choisi parmi des groupements alkylène à chaîne droite et ramifiée comprenant jusqu'à 7 atomes de carbone dans la chaîne principale, qui est non substitué ou substitué par des groupements hydroxy et pouvant comprendre en outre des atomes d'oxygène, d'azote ou de soufre, ou 1 à 3 cycles aromatiques et/ou hétérocycliques ; les atomes d'oxygène, d'azote et de soufre étant présents sous forme de groupements éther, thioéther, sulfoxyde, sulfone, sulfonium, alkylamine ou alcénylamine, de groupements benzylamine, oxyde d'amine, ammonium quaternaire, amide, imide, alcool, ester, et/ou uréthanne.
   b) les polymères de formule :

      -D-X-D-X- (XII)

      dans laquelle D désigne un groupement : 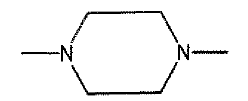 et X désigne le symbole E ou E', où X désigne E' au moins une fois, E a la signification indiquée ci-dessus et E' est un groupement bivalent choisi parmi des groupements alkylène à chaîne droite et ramifiée ayant jusqu'à 7 atomes de carbone dans la chaîne principale, qui est non substitué ou substitué par un ou plusieurs groupements hydroxy et comprenant un ou plusieurs atomes d'azote, l'atome d'azote étant substitué par une chaîne alkyle éventuellement interrompue par un atome d'oxygène et comprenant obligatoirement un ou plusieurs groupements fonctionnels carboxy et un ou plusieurs groupements fonctionnels hydroxy et où le polymère de formule (XII) est bétaïnisé par réaction avec l'acide chloroacétique ou chloroacétate de sodium.
(9) Les copolymères alkyl(C₁-C₅)vinyléther/anhydride maléïque qui sont modifiés partiellement par semiamidification avec une N,N-dialkylaminoalkylamine telle que la N,N-diméthylaminopropylamine, ou par semiestérification avec une N,N-dialcanolamine. Ces copolymères peuvent également comprendre d'autres comonomères vinyliques tels que le vinylcaprolactame.

Dans un mode de mise en oeuvre, les polymères amphotères selon l'invention sont choisis dans la famille (3), comme les copolymères dont le nom CTFA (4e éd. 1991) est copolymère de méthacrylate d'octylacrylamide/d'acrylate/de butylamino éthyle, tels que les produits vendus sous les noms Amphomer, Amphomer LV 71 ou Lovocryl 47 par la société National Starch et la famille (4), tels que le copolymère de méthacrylate de méthyle/diméthyl-carboxyméthylammonio éthyl-méthacrylate de méthyle vendu par exemple sous le nom Diaformer Z301 par la société Sandoz.

Les polymères non ioniques utilisables selon la présente invention sont choisis par exemple parmi :
- les homopolymères de vinylpyrrolidone ;
- les copolymères de vinylpyrrolidone et d'acétate de vinyle ;
- les polyalkyloxazolines telles que les polyéthyloxazolines vendues par la société Dow Chemical sous les noms PEOX 50 000, PEOX 200 000 et PEOX 500 000 ;
- les homopolymères d'acétate de vinyle tels que le produit vendu sous le nom Appretan EM par la société Hoechst ou le produit vendu sous le nom Rhodopas A 012 par la société Rhodia Chimie ;
- les copolymères d'acétate de vinyle et d'ester acrylique tels que le produit vendu sous le nom Rhodopas AD 310 par Rhodia Chimie;
- les copolymères d'acétate de vinyle et d'éthylène tels que le produit vendu sous le nom Appretan TV par la société Hoechst ;
- les copolymères d'acétate de vinyle et d'ester maléïque, par exemple de maléate de dibutyle tels que le produit vendu sous le nom Appretan MB Extra par la société Hoechst ;
- les copolymères de polyéthylène et d'anhydride maléïque ;
- les homopolymères d'acrylate d'alkyle et les homopolymères de méthacrylate d'alkyle tels que le produit vendu sous le nom Micropearl RQ 750 par la société Matsumoto ou le produit vendu sous le nom Luhydran A 848 S par la société BASF ;
- les copolymères d'esters acryliques tels que par exemple les copolymères de (méth)acrylates d'alkyle, tels que les produits vendus par la société Rohm & Haas sous les noms Primal AC-261 K et Eudragit NE 30 D, par la société BASF sous les noms Acronal 601, Luhydran LR 8833 ou 8845, et par la société Hoechst sous les noms Appretan N 9213 ou N 9212 ;
- les copolymères d'acrylonitrile et d'un monomère non ionique choisis par exemple parmi le butadiène et les (méth)acrylates d'alkyle ; on peut citer les produits vendus sous les noms Nipol LX 531 B par la société Nippon Zeon ou ceux vendus sous le nom CJ0601 B par la société Rohm & Haas ;
- les polyuréthanes tels que les produits vendus sous les noms Acrysol RM 1020 ou Acrysol RM 2020 par la société Rohm & Haas, et les produits Uraflex XP 401 UZ et Uraflex XP 402 UZ par la société DSM Resins ;
- les copolymères d'acrylate d'alkyle et d'uréthanne tels que le produit 8538-33 par la société National Starch ;
- les polyamides tels que le produit Estapor LO 11 vendu par la société Rhodia Chimie ;
- les gommes de guar non ioniques chimiquement modifiées ou non modifiées.

Les gommes de guar non ioniques non modifiées sont par exemple les produits vendus sous le nom Vidogum GH 175 par la société Unipectine et sous le nom Jaguar C par la société Rhodia Chimie.

Les gommes de guar non ioniques modifiées utilisables selon l'invention sont par exemple modifiées par des groupements hydroxyalkyle en C₁-C₆. On peut citer à titre d'exemples les groupements hydroxyméthyle, hydroxyéthyle, hydroxypropyle et hydroxybutyle.

Ces gommes de guar sont bien connues dans la technique antérieure et peuvent être préparées par exemple en faisant réagir des oxydes d'alcène correspondants tels que par exemple des oxydes de propylène, avec la gomme de guar de façon à obtenir une gomme de guar modifiée par des groupements hydroxypropyle.

De telles gommes de guar non ioniques éventuellement modifiées par des groupements hydroxyalkyle sont par exemple vendues sous les noms commerciaux Jaguar HP8, Jaguar HP60, Jaguar HP120, Jaguar DC 293, et Jaguar HP 105 par la société Rhodia Chimie et sous le nom Galactosol 4H4FD2 par la société Aqualon.

Les groupements alkyles des polymères non ioniques comprennent de 1 à 6 atomes de carbone, sauf mention contraire.

Selon un autre mode de mise ne oeuvre de l'invention, l'agent conditionneur est une silicone.

Dans tout ce qui suit, on entend désigner par silicone, en conformité avec l'acceptation générale, tous polymères ou oligomères organosiliciés à structure linéaire ou cyclique, ramifiée ou réticulée, de poids moléculaire variable, obtenus par polymérisation et/ou par polycondensation de silanes convenablement fonctionnalisés, et constitués pour l'essentiel par une répétition de motifs principaux dans lesquels les atomes de silicium sont reliés entre eux par des atomes d'oxygène (liaison siloxane -Si-O-Si-), des radicaux hydrocarbonés éventuellement substitués, étant directement liés par l'intermédiaire d'un atome de carbone sur lesdits atomes de silicium. Les radicaux hydrocarbonés les plus courants sont les radicaux alkyles, notamment en C₁-C₁₀, et en particulier méthyle, les radicaux fluoroalkyles dont la partie alkyle est en C₁-C₁₀, les radicaux aryles et en particulier phényle.

Les silicones qui peuvent être utilisées dans la composition selon l'invention, peuvent être volatiles ou non, solubles ou insolubles dans la composition. Elles peuvent être en particulier des polyorganosiloxanes insolubles dans la composition de l'invention et se présenter sous forme d'huiles, de cires, de résines ou de gommes.

Les silicones insolubles sont notamment dispersées dans les compositions sous forme de particules ayant généralement une taille moyenne en nombre comprise entre 2 nanomètres et 100 micromètres, de préférence entre 20 nanomètres et 20 micromètres (mesurée avec un granulomètre).

Les polyorganosiloxanes sont définis plus en détail dans l'ouvrage de Walter NOLL "Chemistry and Technology of Silicones" (1968) Academic Press. Ils peuvent être volatiles ou non volatiles.

Lorsqu'elles sont volatiles, les silicones sont plus particulièrement choisies parmi celles possédant un point d'ébullition compris entre 60° C et 260° C, et plus particulièrement encore parmi :
(i) les silicones cycliques comportant de 3 à 7 atomes de silicium et, de préférence, 4 à 5. Il s'agit, par exemple, de l'octaméthylcyclotétra-siloxane commercialisé notamment sous le nom de "VOLATILE SILICONE 7207" par UNION CARBIDE ou "SILBIONE 70045 V 2" par RHODIA, le décaméthylcyclopentasiloxane commercialisé sous le nom de "VOLATILE SILICONE 7158" par UNION CARBIDE, "SILBIONE 70045 V 5" par RHODIA, ainsi que leurs mélanges.
   On peut également citer les cyclocopolymères du type diméthylsiloxane/méthylalkylsiloxane, tel que la "SILICONE VOLATILE FZ 3109" commercialisée par la société UNION CARBIDE, de structure chimique : 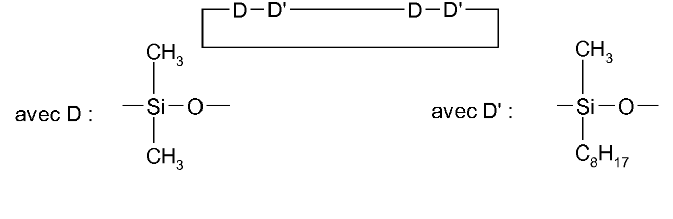 On peut également citer les mélanges de silicones cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'-(hexa-2,2,2',2',3,3'-triméthylsilyloxy) bis-néopentane ;
(ii) les silicones volatiles linéaires ayant 2 à 9 atomes de silicium et possédant une viscosité inférieure ou égale à 5.10⁻⁶m²/s à 25 °C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and toiletries, Vol. 91, Jan. 76, p. 27-32 - TODD & BYERS "Volatile Silicone fluids for cosmetics".

Par silicone non volatile, on entend au sens de la présente invention, toute silicone dont le nombre d'atomes de silicium est supérieur à 7.

Parmi les silicones non volatiles, on peut notamment citer des polyalkylsiloxanes, des polyarylsiloxanes, des polyalkylarylsiloxanes, les gommes et les résines de silicones, les polyorganosiloxanes modifiés par des groupements organofonctionnels, les copolymères blocs linéaires polysiloxane(A)-polyoxyalkylène(B) de type (A-B)ₙ avec n >3 ; les polymères siliconés greffés, à squelette organique non siliconé, constitués d'une chaîne principale organique formée à partir de monomères organiques ne comportant pas de silicone, sur laquelle se trouve greffé, à l'intérieur de ladite chaîne ainsi qu'éventuellement à l'une au moins de ses extrémités, au moins un macromonomère polysiloxane ; les polymères siliconés greffés, à squelette polysiloxanique greffé par des monomères organiques non siliconés, comprenant une chaîne principale de polysiloxane sur laquelle se trouve greffé, à l'intérieur de ladite chaîne ainsi qu'éventuellement à l'une au moins de ses extrémités, au moins un macromonomère organique ne comportant pas de silicone ; ainsi que leurs mélanges.

A titre d'exemples de polyalkylsiloxanes, on peut notamment citer les polydiméthylsiloxanes à groupements terminaux triméthylsilyle ayant une viscosité de 5.10⁻⁶ à 2,5 m²/s à 25°C et de préférence 1.10⁻⁵ à 1 m²/s. La viscosité des silicones est par exemple mesurée à 25°C selon la norme ASTM 445 Appendice C.

Parmi ces polyalkylsiloxanes, on peut citer à titre non limitatif les produits commerciaux suivants :
- les huiles SILBIONE des séries 47 et 70 047 ou les huiles MIRASIL commercialisées par RHONE POULENC telles que par exemple l'huile 70 047 V 500 000 ;
- les huiles de la série MIRASIL commercialisées par la société Rhodia ;
- les huiles de la série 200 de la société DOW CORNING telles que plus particulièrement la DC200 de viscosité 60 000 cSt ;
- les huiles VISCASIL de GENERAL ELECTRIC et certaines huiles des séries SF (SF 96, SF 18) de GENERAL ELECTRIC.

On peut également citer les polydiméthylsiloxanes à groupements terminaux diméthylsilanol (Dimethiconol selon la dénomination CTFA) tels que les huiles de la série 48 de la société Rhodia.

Dans cette classe de polyalkylsiloxanes, on peut également citer les produits commercialisés sous les dénominations "ABIL WAX 9800 et 9801" par la société GOLDSCHMIDT qui sont des polyalkyl (C₁-C₂₀) siloxanes.

Les polyalkylarylsiloxanes peuvent être notamment choisis parmi les polydiméthyl-méthylphénylsiloxanes, les polydiméthyl-diphénylsiloxanes linéaires et/ou ramifiés de viscosité de 1.10⁻⁵ à 5.10⁻² m²/s à 25°C.

Parmi ces polyalkylarylsiloxanes, on peut citer à titre d'exemple les produits commercialisés sous les dénominations suivantes :
- les huiles SILBIONE de la série 70 641 de RHODIA,
- les huiles des séries RHODORSIL 70 633 et 763 de RHODIA ;
- l'huile DOW CORNING 556 COSMETIC GRAD FLUID de DOW CORNING ;
- les silicones de la série PK de BAYER comme le produit PK20 ;
- les silicones des séries PN, PH de BAYER comme les produits PN1000 et PH1000 ;
   certaines huiles des séries SF de GENERAL ELECTRIC telles que SF 1023, SF 1154, SF 1250, SF 1265.

Les gommes de silicone utilisables dans la composition selon l'invention sont notamment des polydiorganosiloxanes ayant des masses moléculaires moyennes en nombre élevées comprises entre 200 000 et 1 000 000, utilisés seuls ou en mélange dans un solvant. Ce solvant peut être choisi parmi les silicones volatiles, les huiles polydiméthylsiloxanes (PDMS), les huiles polyphénylméthylsiloxanes (PPMS), les isoparaffines, les polyisobutylènes, le chlorure de méthylène, le pentane, le dodécane, le tridécanes ou leurs mélanges.

On peut plus particulièrement citer les produits suivants :
- les gommes de polydiméthylsiloxane,
- les gommes de polydiméthylsiloxanes/méthylvinylsiloxane,
- les gommes de polydiméthylsiloxane/diphénylsiloxane,
- les gommes de polydiméthylsiloxane/phénylméthylsiloxane,
- les gommes de polydiméthylsiloxane/diphénylsiloxane/méthylvinylsiloxane.

Peuvent également être employés des mélanges de silicones tels que :
- les mélanges formés à partir d'une gomme de polydiméthylsiloxane hydroxylée en bout de chaîne (dénommé diméthiconol selon la nomenclature du dictionnaire CTFA) et d'un polydiméthylsiloxane cyclique (dénommé cyclométhicone selon la nomenclature du dictionnaire CTFA) tel que le produit Q2 1401 commercialisé par la société DOW CORNING ;
- les mélanges formés à partir d'une gomme polydiméthylsiloxane avec une silicone cyclique tel que le produit SF 1214 Silicone Fluid de la société GENERAL ELECTRIC, ce produit est une gomme SF 30 correspondant à une diméthicone, ayant un poids moléculaire moyen en nombre de 500 000 solubilisée dans l'huile SF 1202 Silicone Fluid correspondant au décaméthylcyclopentasiloxane ;
- les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 m²/s et d'une huile SF 96 d'une viscosité de 5.10⁻⁶m²/s. Ce produit comporte de préférence 15 % de gomme SE 20 et 85 % d'une huile SF 96.

Les résines de polyorganosiloxanes utilisables dans la composition selon l'invention sont des systèmes siloxaniques réticulés renfermant les unités : R₂SiO_{2/2}, R₃SiO_{1/2}, RSiO_{3/2} et SiO_{4/2} dans lesquelles R représente un groupement hydrocarboné possédant 1 à 16 atomes de carbone ou un groupement phényle.

Parmi ces produits, ceux particulièrement préférés sont ceux dans lesquels R désigne un radical alkyle inférieur en C₁-C₄, plus particulièrement méthyle, ou un radical phényle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl/triméthyl siloxane.

On peut également citer les résines du type triméthylsiloxysilicate commercialisées notamment sous les dénominations X22-4914, X21-5034 et X21-5037 par la société SHIN-ETSU.

Les silicones organomodifiées utilisables dans la composition selon l'invention sont des silicones telles que définies précédemment et comportant dans leur structure un ou plusieurs groupements organofonctionnels fixés par l'intermédiaire d'un groupe hydrocarboné.

Parmi les silicones organomodifiées, on peut citer les polyorganosiloxanes comportant :
- des groupements polyéthylèneoxy et/ou polypropylèneoxy comportant éventuellement des groupements alkyle en C₆-C₂₄ tels que les produits dénommés diméthicone-copolyol commercialisé par la société DOW CORNING sous la dénomination DC 1248 ou les huiles SILWET^{®} L 722, L 7500, L 77, L 711 de la société UNION CARBIDE et l'alkyl(C₁₂)-méthicone-copolyol commercialisée par la société DOW CORNING sous la dénomination Q2 5200 ;
- des groupements thiols, comme les produits commercialisés sous les dénominations "GP 72 A" et "GP 71" de GENESEE ;
- des groupements alcoxylés, comme le produit commercialisé sous la dénomination "SILICONE COPOLYMER F-755" par SWS SILICONES et ABIL WAX^{®} 2428, 2434 et 2440 par la société GOLDSCHMIDT ;
- des groupements hydroxylés, comme les polyorganosiloxanes à fonction hydroxyalkyle décrits dans la demande de brevet français FR-A-85 16334 ;
- des groupements acyloxyalkyle tels que, par exemple, les polyorganosiloxanes décrits dans le brevet US-A-4957732.
- des groupements anioniques du type acide carboxylique comme, par exemple, dans les produits décrits dans le brevet EP 186 507 de la société CHISSO CORPORATION, ou du type alkyl-carboxylique comme ceux présents dans le produit X-22-3701E de la société SHIN-ETSU ; 2-hydroxyalkylsulfonate ; 2-hydroxyalkylthiosulfate tels que les produits commercialisés par la société GOLDSCHMIDT sous les dénominations "ABIL^{®} S201" et "ABIL^{®} S255".
- des groupements hydroxyacylamino, comme les polyorganosiloxanes décrits dans la demande EP 342 834. On peut citer, par exemple, le produit Q2-8413 de la société DOW CORNING.

De préférence, la silicone est une silicone aminée.

Par silicone aminée, on entend au sens de la présente invention, toute silicone comportant au moins une fonction amine primaire, secondaire, tertiaire ou un groupement ammonium quaternaire.

Les silicones aminées qui peuvent être utilisées dans la composition selon la présente invention sont choisies parmi :
(a) les composés répondant à la formule (XIII) suivante :

   (R¹)ₐ(T)₃₋ₐ-Si[OSi(T)₂]ₙ-[OSi(T)_{b}(R¹)_{2-b}]ₘ-OSi(T)₃₋ₐ-(R¹)ₐ (XIII)
dans laquelle,
T est un atome d'hydrogène, ou un radical phényle, hydroxyle (-OH), ou alkyle en C₁-C₈, et de préférence méthyle ou alcoxy en C₁-C₈, de préférence méthoxy,
a désigne le nombre 0 ou un nombre entier de 1 à 3, et de préférence 0,
b désigne 0 ou 1, et en particulier 1,
m et n sont des nombres tels que la somme (n + m) peut varier notamment de 1 à 2 000 et en particulier de 50 à 150, n pouvant désigner un nombre de 0 à 1 999 et notamment de 49 à 149 et m pouvant désigner un nombre de 1 à 2 000, et notamment de 1 à 10 ;
R₁ est un radical monovalent de formule -C_{q}H_{2q}L dans laquelle q est un nombre de 2 à 8 et L est un groupement aminé éventuellement quaternisé choisi parmi les groupements :

-N(R²)-CH₂-CH₂-N(R²)₂ ;

-N(R²)₂ ; -N⁺(R²)₃ Q⁻ ;

-N⁺(R²) (H)2 Q⁻ ;

-N⁺(R²)₂HQ⁻ ;

-N(R²)-CH₂-CH₂-N⁺(R²)(H)₂ Q⁻,

dans lesquels R² peut désigner un atome d'hydrogène, un phényle, un benzyle, ou un radical hydrocarboné saturé monovalent, par exemple un radical alkyle en C₁-C₂₀, et Q- représente un ion halogénure tel que par exemple fluorure, chlorure, bromure ou iodure.

En particulier, les silicones aminées correspondant à la définition de la formule (XIII) sont choisies parmi les composés correspondant à la formule suivante (XIV) : 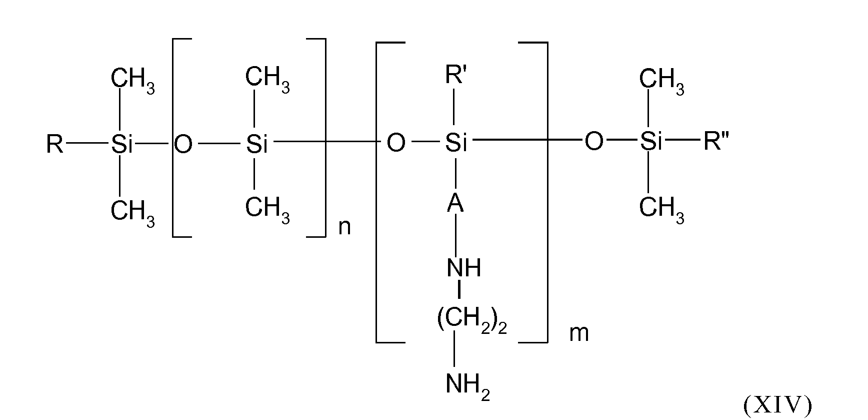 dans laquelle R, R', R", identiques ou différents, désignent un radical alkyle en C₁-C₄, de préférence CH₃ ; un radical alcoxy en C₁-C₄, de préférence méthoxy ; ou OH ; A représente un radical alkylène, linéaire ou ramifié, en C₃-C₈, de préférence en C₃-C₆; m et n sont des nombres entiers dépendant du poids moléculaire et dont la somme est comprise entre 1 et 2000.

Selon une première possibilité, R, R', R", identiques ou différents, représentent un radical alkyle en C₁-C₄ ou hydroxyle, A représente un radical alkylène en C₃ et m et n sont tels que la masse moléculaire moyenne en poids du composé est comprise entre 5 000 et 500 000 environ. Les composés de ce type sont dénommés dans le dictionnaire CTFA, "amodiméthicone".

Selon une deuxième possibilité, R, R', R", identiques ou différents, représentent un radical alcoxy en C₁-C₄ ou hydroxyle, l'un au moins des radicaux R ou R" est un radical alcoxy et A représente un radical alkylène en C₃. Le rapport molaire hydroxy / alcoxy est de préférence compris entre 0,2/1 et 0,4/1 et avantageusement égal à 0,3/1. Par ailleurs, m et n sont tels que la masse moléculaire moyenne en poids du composé est comprise entre 2000 et 10⁶. Plus particulièrement, n est compris entre 0 et 999 et m est compris entre 1 et 1000, la somme de n et m étant comprise entre 1 et 1000.

Dans cette catégorie de composés, on peut citer entre autres, le produit Belsil®ADM 652, commercialisée par Wacker.

Selon une troisième possibilité, R, R", différents, représentent un radical alcoxy en C₁-C₄ ou hydroxyle, l'un au moins des radicaux R, R" est un radical alcoxy, R' représente un radical méthyle et A représente un radical alkylène en C₃. Le rapport molaire hydroxy / alcoxy est de préférence compris entre 1/0,8 et 1/1,1, et avantageusement est égal à 1/0,95. Par ailleurs, m et n sont tels que la masse moléculaire moyenne en poids du composé est comprise entre 2000 et 200000. Plus particulièrement, n est compris entre 0 et 999 et m est compris entre 1 et 1000, la somme de n et m étant comprise entre 1 et 1000.

Plus particulièrement, on peut citer le produit FluidWR® 1300, commercialisé par Wacker.

La masse moléculaire de ces silicones est déterminée par chromatographie par perméation de gel (température ambiante, étalon polystyrène ; colonnes µ styragem ; éluant THF ; débit de 1 mm/m ; on injecte 200 µl d'une solution à 0,5 % en poids de silicone dans le THF et l'on effectue la détection par réfractométrie et UV-métrie).

Un produit correspondant à la définition de la formule (XIII) est en particulier le polymère dénommé dans le dictionnaire CTFA "triméthylsilylamodiméthicone", répondant à la formule (XV) suivante: 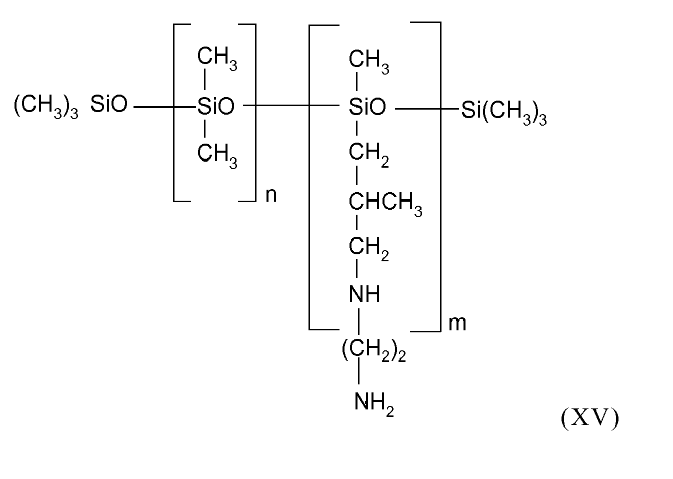 dans laquelle n et m ont les significations données ci-dessus conformément à la formule (XIV).

De tels composés sont décrits par exemple dans EP 95238; un composé de formule (XIII) est par exemple vendu sous la dénomination Q2-8220 par la société OSI.

### (b) les composés répondant à la formule (XVI) suivante :

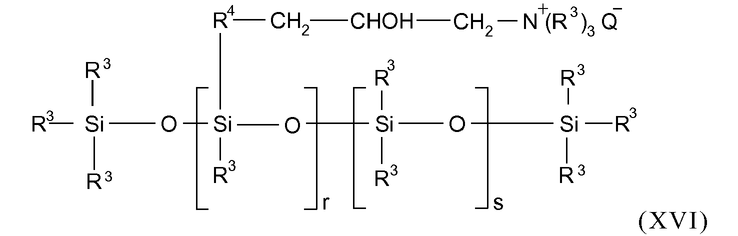

dans laquelle,
R³ représente un radical hydrocarboné monovalent en C₁-C₁₈, et en particulier un radical alkyle en C₁-C₁₈, ou alcényle en C₂-C₁₈, par exemple méthyle ;
R⁴ représente un radical hydrocarboné divalent, notamment un radical alkylène en C₁-C₁₈ ou un radical alkylèneoxy divalent en C₁-C₁₈ , par exemple en C₁-C₈,
Q⁻ est un ion halogénure, notamment chlorure ;
r représente une valeur statistique moyenne de 2 à 20 et en particulier de 2 à 8 ;
s représente une valeur statistique moyenne de 20 à 200 et en particulier de 20 à 50.

De tels composés sont décrits plus particulièrement dans le brevet US 4185087.

Un composé entrant dans cette classe est celui vendu par la Société Union Carbide sous la dénomination "Ucar Silicone ALE 56".

### c) les silicones ammonium quaternaire de formule (XVII) :

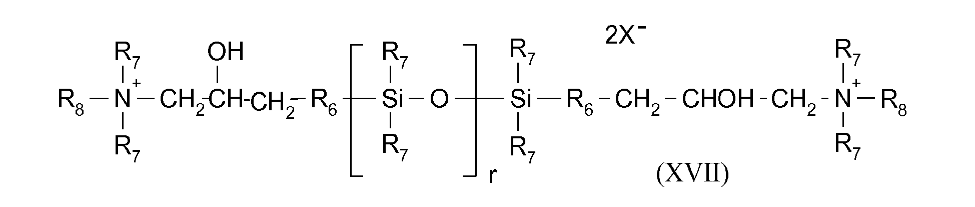

dans laquelle :
R₇, identiques ou différents, représentent un radical hydrocarboné monovalent ayant de 1 à 18 atomes de carbone, et en particulier un radical alkyle en C₁-C₁₈, un radical alcényle en C₂-C₁₈ ou un cycle comprenant 5 ou 6 atomes de carbone, par exemple méthyle ;
R₆ représente un radical hydrocarboné divalent, notamment un radical alkylène en C₁-C₁₈ ou un radical alkylèneoxy divalent en C₁-C₁₈, par exemple en C₁-C₈ relié au Si par une liaison SiC;
R₈, identiques ou différents, représentent un atome d'hydrogène, un radical hydrocarboné monovalent ayant de 1 à 18 atomes de carbone, et en particulier un radical alkyle en C₁-C₁₈, un radical alcényle en C₂-C₁₈ , un radical -R₆-NHCOR₇ ;
X- est un anion tel qu'un ion halogénure, notamment chlorure ou un sel d'acide organique (acétate ...);
r représente une valeur statistique moyenne de 2 à 200 et en particulier de 5 à 100 ;
Ces silicones sont par exemple décrites dans la demande EP-A-0530974.

Selon l'invention, on peut également utiliser des polymères du type silicone greffée comprenant une portion polysiloxane et une portion comprenant une chaîne organique non siliconée, l'une des deux portions constituant la chaîne principale du polymère et l'autre étant greffée sur la chaîne principale. Ces polymères sont divulgués, par exemple, dans les documents EP A 0 412 704, EP-A-0 412 707, EP-A-0 640 105, WO 95/00578, EP-A-0 582 152 et WO 93/23009 et les brevets U.S. n° 4 693 935, 4 728 571 et 4 972 037. Ces polymères sont par exemple anioniques ou non ioniques.

De tels polymères sont par exemple des copolymères susceptibles d'être obtenus par polymérisation radicalaire à partir du mélange de monomères comprenant :
a) environ 50 % à environ 90 % en poids d'acrylate de tert-butyle ;
b) 0 % à environ 40 % en poids d'acide acrylique ;
c) environ 5 % à environ 40 % en poids de macromère siliconé de formule : 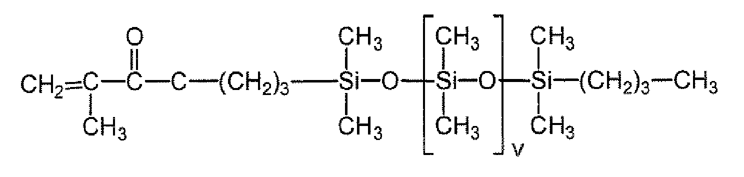
dans laquelle v est un nombre allant de 5 à 700 ; les pourcentages en poids étant calculés par rapport au poids total des monomères.

D'autres exemples de polymères siliconés greffés sont notamment des polydiméthylsiloxanes (PDMS) sur lesquels sont greffés, par l'intermédiaire d'une chaîne de raccordement de type thiopropylène, des motifs polymères mixtes du type acide poly(méth)acrylique et du type poly(méth)acrylate d'alkyle et des polydiméthylsiloxanes (PDMS) sur lesquels sont greffés, par l'intermédiaire d'une chaîne de raccordement de type thiopropylène, des motifs polymères du type poly(méth)acrylate d'isobutyle.

Il est également possible d'utiliser, comme polymères, des polyuréthanes fonctionnalisés ou non et siliconés ou non.

Des exemples de polyuréthanes utiles incluent ceux divulgués dans les brevets EP 0 751 162, EP 0 637 600, FR 2 743 297, EP 0 648 485, EP 0 656 021, WO 94/03510 et EP 0 619 111.

Les polymères conditionneurs siliconés ou non peuvent être utilisés sous forme solubilisée ou peuvent être sous forme de dispersions de particules solides ou liquides (latex ou pseudolatex).

Selon le premier mode de réalisation, la composition peut également comprendre un ou plusieurs tensioactifs choisis parmi les tensioactifs anioniques, amphotères, non ioniques, cationiques, et des mélanges de ceux-ci. Le ou les tensioactifs sont présents en une quantité allant de 0,1 % à 60 % en poids, par exemple de 1 % à 40 %, en outre de 5 % à 30 %, par rapport au poids total de la composition. Dans une variante, la composition est un shampooing dans lequel le ou les tensioactifs sont présents en une quantité allant de 5 % à 30 % par rapport au poids total de la composition. Dans une autre variante, la composition est un après-shampooing particulier, dans lequel un ou plusieurs tensioactifs cationiques sont présents en une quantité allant de 0,1 % à 15 % par rapport au poids total de la composition et de préférence de 0,5 à 5% en poids.

Les tensioactifs représentatifs convenant à la mise en oeuvre de la présente invention sont par exemple les suivants :

### (i) Tensioactif(s) anionique(s) :

Des exemples de tensioactifs anioniques pouvant être utilisés seuls ou en mélange incluent les sels (tels que les sels alcalins, notamment les sels de sodium, les sels d'ammonium, les sels d'amine, les sels d'aminoalcool et les sels de magnésium) de composés (tels que les alkylsulfates, les alkyléthersulfates, les alkylamidoéthersulfates, les alkylarylpolyéthersulfates, les monoglycéride-sulfates, les alkylsufonates, les alkylphosphates, les alkylamidesulfonates, les alkylarylsufonates, les α-oléfine-sulfonates, les paraffine-sulfonates, les alkylsulfosuccinates, les alkyléthersulfosuccinates, les alkylamidesulfosuccinates, les alkylsulfosuccinamates, les alkylsulfoacétates, les alkylétherphosphates, les acylsarcosinates, les acyliséthionates et les N-acyltaurates). Dans un mode de mise en oeuvre, les groupements alkyle et acyle contiennent d'environ 8 à environ 24 atomes de carbone et les groupements aryle sont choisis parmi les groupements phényle et benzyle.

Parmi les tensioactifs anioniques pouvant également être utilisés, on peut également citer les sels d'acide gras (tels que les sels des acides oléïque, ricinoléïque, palmitique, stéarique, les acides d'huile de coprah et d'huile de coprah hydrogénée) ; et les acyllactylates dont le groupement acyle contient de 8 à 20 atomes de carbone. On peut également utiliser des tensioactifs faiblement anioniques, comme les acides alkyl-D-galactosiduroniques et leurs sels ainsi que les acides alkyl(C₆-C₂₄)éthercarboxyliques polyoxyalkylénés, les acides alkyl(C₆-C₂₄)aryléthercarboxyliques polyoxyalkylénés, les acides alkyl(C₆-C₂₄)amidoéthercarboxyliques polyoxyalkylénés et leurs sels, comme ceux contenant de 2 à 50 groupements oxyde d'éthylène, et leurs mélanges.

Dans un mode de mise en oeuvre, le(s) tensioactif(s) anionique(s) peut (peuvent) être choisi(s) parmi les sels d'alkylsulfate, les sels d'alkyléthersulfate, et leurs mélanges. Dans un autre mode de mise en oeuvre, le tensioactif anionique est choisi parmi les lauryléthersulfates de sodium, d'ammonium ou de magnésium.

### (ii) Tensioactif(s) non ionique(s) :

Les tensioactifs non ioniques sont par exemple décrits dans « Handbook of Surfactants » par M.R. Porter, édité par Blackie & Son (Glasgow et Londres, 1991, pages 116 - 178). Des exemples de tensioactifs non ioniques pouvant être utilisés seuls ou en mélange incluent, les alkylphénols, les α-diols et les alcools ; qui ont tous une chaîne grasse contenant par exemple de 8 à 18 atomes de carbone et qui ont été polyéthoxylés, polypropoxylés, et/ou polyglycérolés, où le nombre de groupements d'oxyde d'éthylène ou d'oxyde de propylène varie de 2 à 50 et le nombre de groupements glycérol varie de 2 à 30. On peut également citer les copolymères d'oxyde d'éthylène et d'oxyde de propylène, les condensats d'oxyde d'éthylène et d'oxyde de propylène sur les alcools gras ; les amides gras polyéthoxylés (tels que ceux ayant de 2 à 30 moles d'oxyde d'éthylène) ; les amides gras polyglycérolés contenant en moyenne 1 à 5, par exemple de 1,5 à 4 groupements glycérol ; les amines grasses polyéthoxylées (telles que celles ayant de 2 à 30 moles d'oxyde d'éthylène) ; les esters d'acide gras du sorbitane oxyéthylénés ayant de 2 à 30 moles d'oxyde d'éthylène ; les esters d'acides gras du sucrose ; les esters d'acides gras de polyéthylèneglycol ; les alkylpolyglycosides ; les dérivés de N-alkylglucamine ; et les oxydes d'amines tels que les oxydes d'alkyl(C₁₀-C₁₄)amines et les oxydes de N-acylaminopropylmorpholine. Dans un mode de mise en oeuvre, les tensioactifs non ioniques sont choisis parmi les alkylpolyglycosides.

### (iii) Tensioactif(s) amphotère(s) :

Les tensioactifs amphotères peuvent être choisis par exemple parmi des dérivés d'amines secondaires ou tertiaires aliphatiques dans lesquels le radical aliphatique est choisi parmi des chaînes linéaires et ramifiées contenant 8 à 22 atomes de carbone et contenant au moins un groupement anionique hydrosoluble (par exemple des groupements carboxylate, sulfonate, sulfate, phosphate et phosphonate). Des exemples convenables incluent les alkyl(C₈-C₂₀)bétaïnes, les sulfobétaïnes, les alkyl(C₈-C₂₀)amidoalkyl(C₁-C₆)bétaïnes et les alkyl(C₈-C₂₀)amidoalkyl(C₁-C₆)sulfo bétaïnes.

Parmi les dérivés d'amines, on peut citer les produits de formules (A) et (B) :

R₂-CONHCH₂CH₂-N⁺(R₃)(R₄)(CH₂COO⁻) (A)

dans laquelle R₂ est choisi parmi des groupements alkyle issus d'un acide R₂-COOH présent dans l'huile de coprah hydrolysée, des groupements heptyle, nonyle et undécyle, R₃ est choisi parmi des groupements β-hydroxyéthyle, et R₄ est choisi parmi des groupements carboxyméthyle ;
et

R₅-CONHCH₂CH₂-N (B) (C) (B)

dans laquelle B est choisi parmi -CH₂CH₂OX', C est choisi parmi -(CH₂)_{z}-Y', avec z = 1 ou 2 ; X' est choisi parmi les groupements -CH₂CH₂-COOH et l'hydrogène ; Y' est choisi parmi les groupements -COOH et -CH₂-CHOH-SO₃H ; R₅ est choisi parmi les radicaux alkyle d'un acide R₉-COOH présent dans l'huile de coprah et dans l'huile de lin hydrolysée, les radicaux alkyle (tels que les radicaux alkyle en C₇, C₉, C₁₁ et C₁₃), et les radicaux alkyle en C₁₇ (tels que la forme iso et les radicaux C₁₇ insaturés).

Ces composés sont classés dans le dictionnaire CTFA, 5ème édition, 1993, sous les noms disodium cocoamphodiacetate, disodium lauroamphodiacetate, disodium caprylamphodiacetate, disodium capryloamphodiacetate, disodium cocoamphodipropionate, disodium lauroamphodipropionate, disodium caprylamphodipropionate, disodium capryloamphodipropionate, lauroamphodipropionic acid, et cocoamphodipropionic acid. Un exemple utile peut être le cocoamphodiacétate vendu sous le nom commercial Miranol C2M concentré par la société Rhodia Chimie.

### (iv) Tensioactif(s) cationique(s)

Les tensioactifs cationiques peuvent être choisis parmi :
A) les sels d'ammonium quaternaires de formule (XVIII) ci-dessous : 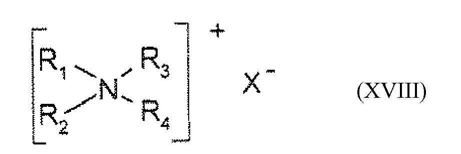 dans laquelle X⁻ est un anion choisi parmi des halogénures (tels que chlorure, bromure et iodure) et les alkyl(C₂-C₆)sulfates (tels que les méthylsulfates), les phosphates, les alkyl- et alkylarylsulfonates, les anions issus d'acides organiques (tels que l'acétate et le lactate), et soit :
   (i) R₁, R₂ et R₃ sont choisis indépendamment parmi des groupements aliphatiques linéaires et ramifiés contenant de 1 à 4 atomes de carbone et des groupements aromatiques (tels qu'aryles et alkylaryles). Les groupements aliphatiques peuvent comprendre des hétéroatomes tels que par exemple l'oxygène, l'azote et le soufre, et des halogènes. Les groupements aliphatiques peuvent être choisis par exemple parmi les groupements alkyle, alcoxy, cétyle, stéaryle et alkylamide. R₄ peut être choisi parmi des groupements alkyle linéaires et ramifiés contenant de 12 à 30 atomes de carbone. Dans un mode de mise en oeuvre, le tensioactif cationique est choisi parmi les sels de béhényltriméthylammonium (par exemple chlorure) ; soit
   (ii) R₁ et R₂ sont choisis indépendamment parmi des groupements aliphatiques linéaires et ramifiés contenant de 1 à 4 atomes de carbone et des groupements aromatiques dont les aryles et les alkylaryles. Les groupements aliphatiques peuvent comprendre un
   ou plusieurs halogènes et hétéroatomes tels que par exemple l'oxygène, l'azote et le soufre. Les groupements aliphatiques peuvent être choisis par exemple parmi les groupements alkyle, alcoxy, alkylamide et hydroxyalkyle contenant d'environ 1 à 4 atomes de carbone. R₃ et R₄ sont choisis indépendamment parmi les groupements alkyle linéaires et ramifiés contenant de 12 à 30 atomes de carbone, lesdits groupements peuvent comprendre au moins une fonction provenant des fonctions esters et amides. Dans un mode de mise en oeuvre, R₃ et R₄ peuvent être choisis parmi les groupements alkyl(C₁₂-C₂₂)amidoalkyle(C₂-C₆) et alkyl(C₁₂-C₂₂)acétate.
   Dans un mode de mise en oeuvre, le tensioactif cationique est choisi parmi les sels de stéaramidopropyldiméthyl(myristylacétate)ammonium (par exemple chlorure). Dans un autre mode de mise en oeuvre, le tensioactif cationique est choisi parmi le chlorure de palmitamidopropyltrimonium, qui est vendu sous le nom Varisoft PTC par Degussa Goldschmidt ;
B) les sels d'ammonium quaternaires de l'imidazolinium, comme, par exemple, celui de formule (XIX) : 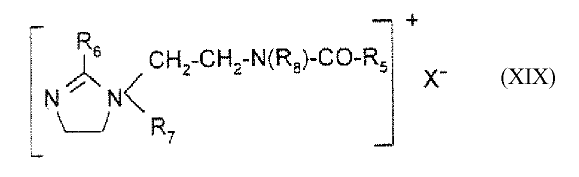 dans laquelle R₅ est choisi parmi les groupements alcényle et alkyle contenant de 8 à 30 atomes de carbone, tels que, par exemple, les dérivés d'acides gras du suif ; R₆ est choisi parmi l'hydrogène, et les groupements alcényle et alkyle contenant de 8 à 30 atomes de carbone ; R₇ est choisi parmi les groupements alkyle en C₁-C₄ ; R₈ est choisi parmi l'hydrogène et les groupements alkyle en C₁-C₄ ; et X⁻ est un anion choisi parmi les halogénures, les phosphates, les acétates, les lactates, les alkylsulfates, les alkylsulfonates et les alkylarylsulfonates. Dans un mode de mise en oeuvre, R₅ et R₆ sont choisis indépendamment parmi les groupements alcényle et alkyle contenant de 12 à 21 atomes de carbone, tels que, par exemple, les dérivés d'acides gras du suif ; R₇ est un groupement méthyle ; et R₈ est un hydrogène. Des exemples incluent Quaternium-27 (CTFA 1997) et Quaternium-83 (CTFA 1997), qui sont vendus sous les noms « Rewoquat » W75, W90, W75PG et W75HPG par la société Witco ;
C) les sels de diammonium quaternaires de formule (XX) : 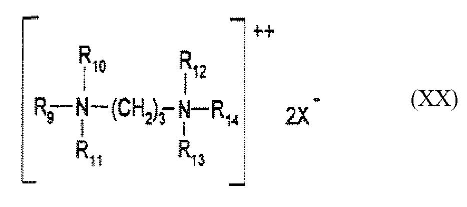 dans laquelle R₉ est choisi parmi les groupements aliphatiques contenant d'environ 16 à environ 30 atomes de carbone ; R₁₀, R₁₁, R₁₂, R₁₃ et R₁₄ sont choisis indépendamment parmi l'hydrogène et les groupements alkyle contenant de 1 à 4 atomes de carbone ; et X⁻ est un anion choisi parmi les halogénures, les acétates, les phosphates, les nitrates et les méthylsulfates. De tels sels de diammonium quaternaires comprennent le dichlorure de propanesuifdiammonium ; et
D) les sels d'ammonium quaternaires contenant au moins une fonction ester de formule (XXI) ci-dessous : 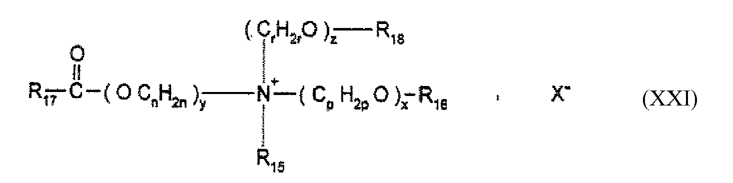
dans laquelle R₁₅ est choisi parmi les groupements alkyle en C₁-C₆, hydroxyalkyle en C₁-C₆ et dihydroxyalkyle ; R₁₆ est choisi parmi les groupements R₁₉-CO-, les groupements R₂₀ hydrocarbonés en C₁-C₂₂ linéaires et ramifiés, saturés et insaturés, et l'hydrogène ; R₁₈ est choisi parmi les groupements R₂₁-CO-, les groupements R₂₂ hydrocarbonés en C₁-C₆ linéaires et ramifiés, saturés et insaturés, et l'hydrogène ; R₁₇, R₁₉ et R₂₁ sont choisis indépendamment parmi les groupements hydrocarbonés en C₇-C₂₁, linéaires et ramifiés, saturés et insaturés ; n, p et r sont des entiers allant indépendamment de 2 à 6 ; y est un entier allant de 1 à 10 ; x et z sont des entiers allant indépendamment de 0 à 10 ; X⁻ est choisi parmi des anions simples et complexes, organiques et inorganiques ; sous réserve que la somme x + y + z aille de 1 à 15, que lorsque x est 0, alors R₁₆ désigne R₂₀, et que lorsque z est 0, alors R₁₈ désigne R₂₂.

Dans un mode de mise en oeuvre, R₁₅ est choisi parmi les groupements méthyle et éthyle ; x et y sont égaux à 1 ; z est égal à 0 ou 1 ; n, p et r sont égaux à 2 ; R₁₆ est choisi parmi les groupements R₁₉-CO-, méthyle, éthyle, et hydrocarbonés en C₁₄-C₂₂, et l'hydrogène ; R₁₇, R₁₉ et R₂₁ sont choisis indépendamment parmi les groupements hydrocarbonés en C₇-C₂₁ linéaires et ramifiés, saturés et insaturés ; R₁₈ est choisi parmi R₂₁-CO- et l'hydrogène.

Des exemples incluent des composés vendus sous les noms Dehyquart par la société Henkel, Stepanquat par la société Stepan, Noxamium par la société Ceca, et Rewoquat WE 18 par la société Rewo-Witco.

Dans un mode de mise en oeuvre, les tensioactifs cationiques sont choisis parmi le chlorure de béhényltriméthylammonium et le chlorure de stéaramidopropylméthyl(myristylacétate)ammonium vendus sous le nom « Ceraphyl 70 » par la société Van Dyk, et Quaternium-27 ou Quaternium-83 vendu par la société Witco.

Dans les compositions conformes à l'invention, on peut utiliser des mélanges de tensioactifs. Par exemple, des mélanges de tensioactifs anioniques et des mélanges de tensioactifs anioniques avec des tensioactifs amphotères et/ou non ioniques sont possibles.

Dans un mode de mise en oeuvre, on choisit un mélange comprenant au moins un tensioactif anionique et au moins un tensioactif amphotère. Le tensioactif anionique peut être choisi par exemple parmi les alkyl(C₁₂-C₁₄)sulfates de sodium, les alkyl(C₁₂-C₁₄)sulfates de triéthanolamine, les alkyl(C₁₂-C₁₄)sulfates d'ammonium, les alkyl(C₁₂-C₁₄)éthersulfates de sodium oxyéthylénés avec 2,2 moles d'oxyde d'éthylène, les alkyl(C₁₂-C₁₄)éthersulfates de triéthanolamine oxyéthylénés avec 2,2 moles d'oxyde d'éthylène, les alkyl(C₁₂-C₁₄)éthersulfates d'ammonium oxyéthylénés avec 2,2 moles d'oxyde d'éthylène, les cocoyliséthionates de sodium et les α-oléfines(C₁₄-C₁₆)sulfonates de sodium. Le tensioactif amphotère peut être choisi, par exemple, parmi les dérivés d'amines dénommés cocoamphodipropionates disodiques et cocoamphopropionates sodiques, tels que ceux vendus par la société Rhodia Chimie sous le nom commercial « Miranol C2M Conc. » sous forme de solution aqueuse à 38 % de matière active, ou sous le nom Miranol C32, et les tensioactifs amphotères du type zwittérionique, dont les alkylbétaïnes, tels que la cocoylbétaïne vendue sous le nom « Dehyton AB 30 » sous forme de solution aqueuse à 32 % de MA par la société Henkel ou la cocoylamidopropylbétaïne. Dans un mode préféré de mise en oeuvre, le mélange comprend la cocoylbétaïne et le lauryléthersulfate de sodium.

Selon un mode de réalisation préféré de l'invention, la composition selon l'invention comprend un ou plusieurs polymères cationiques et/ou une ou plusieurs silicones, et un ou plusieurs tensioactifs.

La composition selon l'invention peut être vaporisable, par exemple par une pompe, ou peut être une composition aérosol sous pression. Elle peut être vaporisable par une valve de distribution contrôlée par une tête de distribution, qui comprend à son tour une buse, qui vaporise la composition aérosol. Une composition vaporisable selon l'invention comprend un solvant approprié. De manière avantageuse, le solvant approprié comprend au moins un solvant choisi parmi l'eau et des alcools inférieurs. Conformément à l'invention, on entend par alcool inférieur un alcool aliphatique en C₁ à C₄, tel que l'éthanol.

Lorsque la composition vaporisable selon l'invention est une composition aérosol, elle comprend en outre une quantité appropriée d'agent propulseur. L'agent propulseur comprend des gaz comprimés ou liquéfiés, qui sont normalement utilisés pour la préparation de compositions aérosol. Les gaz convenables incluent l'air comprimé, le gaz carbonique, l'azote, et les gaz susceptibles d'être solubles dans la composition, tels que l'éther diméthylique, les hydrocarbures fluorés
ou non fluorés, et leurs mélanges.

La présente invention vise en outre un procédé de traitement de fibres kératiniques, notamment les cheveux, dans lequel la composition de coiffage repositionnable selon l'invention, telle que décrite ci-dessus, est appliquée sur les cheveux avant, pendant ou après la mise en forme de la coiffure.

La présente invention vise en outre l'utilisation d'une composition telle que décrite ci-dessus dans une formulation cosmétique de coiffage repositionnable ou pour sa préparation.

La détermination de la capacité d'une composition contenant un copolymère (méth)acrylique selon l'invention à donner un effet repositionnable peut être déterminée par un test in vivo.

Lorsque la composition est sous forme de lotion, par exemple, le test in vivo se déroule comme suit. Les cheveux du modèle sont lavés, puis divisés en deux portions symétriques, les côtés droit et gauche. La composition est appliquée sur un côté de la tête du modèle, puis rincée, alors qu'une composition de référence est appliquée sur l'autre côté de la tête. La composition de référence peut être choisie, par exemple, parmi l'eau, un produit commercial existant, ou une autre composition à l'étude. Le coiffeur sèche et coiffe les deux côtés de la tête. Les deux côtés de la tête sont évalués séparément pour l'effet de coiffage, les propriétés cosmétiques, et l'effet repositionnable. Par exemple, une fois séchés, les cheveux sont brossés dans différentes directions pour enlever la coiffure d'origine. Les cheveux sont alors brossés pour restaurer la coiffure d'origine. Le procédé pour enlever la coiffure, restaurer la coiffure, et évaluer le succès de restauration de la coiffure est répété au moins une fois de plus pour déterminer si la composition est une composition de coiffage repositionnable. Une composition de coiffage repositionnable permet (1) la restauration de la coiffure d'origine après brossage et (2) la création d'une nouvelle coiffure après brossage, qui peut également être restaurée après brossage. Si la composition à évaluer est sous une autre forme, telle qu'un shampooing ou un après-shampooing, le test in vivo peut être modifié de façon appropriée par l'homme de l'art.

Il est entendu que l'homme de l'art pourrait reconnaître que ce n'est pas toutes les formulations qui donneraient un effet repositionnable pour tous les types de cheveu pendant l'essai in vivo et saura comment formuler et évaluer des compositions de coiffage repositionnables en vue des différents paramètres capillaires, tels que la longueur (court contre long), le diamètre (fin contre épais), la structure (frisé contre défrisé), l'état (gras, sec ou normal) ; et si les cheveux sont colorés, décolorés, permanentés ou défrisés. Ainsi, l'essai in vivo peut nécessiter l'essai sur 10-20 individus différents.

### EXEMPLES :

### 1) Préparation d'une émulsion de copolymère (méth)acrylique :

Un mélange de 300 grammes d'acrylate de 2-éthylhexyle (2-EHA), 175 grammes d'acrylate de n-butyle (BA), et 25 grammes de méthacrylate de 2-hydroxyéthyle (HEMA) a été préparé, donnant 500 grammes d'une solution de monomère contenant 60/35/5 parties de 2-EHA/BA/HEMA. On a chargé 50 grammes de l'ensemble de la solution de monomère dans une fiole à résine divisée de deux litres ainsi que 380 grammes d'eau désionisée et 0,5 gramme de RHODACAL DS-10 (tensioactif dodécylbenzènesulfonate de sodium disponible dans le commerce auprès de Rhodia Chimie). La tête a été placée sur la fiole et on a attaché un thermocouple, une entrée d'azote et un agitateur mécanique. Le contenu a été chauffé par des lampes à infrarouge jusqu'à environ 60°C tout en agitant à 350 t/min. On a chargé une solution de 1 gramme d'amorceur persulfate de potassium dans 20 grammes d'eau désionisée, on a fermé la fiole de manière étanche, et on a fait un vide dans la fiole quatre fois, en le cassant à chaque fois avec de l'azote. La fiole a été maintenue à 60°C pendant 20 minutes, puis chauffée à 80°C pendant 10 minutes pour donner un polymère amorce. On a préparé une pré-émulsion à partir des 450 grammes restants de la solution de monomère en la chargeant d'une solution de 4,5 grammes de dodécylbenzènesulfonate de sodium dans 211 grammes d'eau désionisée et en agitant sous l'azote. Cette pré-émulsion a été ajoutée goutte à goutte à la fiole à résine divisée de deux litres contenant le polymère amorce à un débit de 6 grammes par minute. L'addition a duré presque 2 heures. Après l'addition, la vitesse d'agitation a été réduite à 200 t/min et la réaction a été maintenue à 80°C pendant deux heures, puis le latex résultant a été filtré sur une toile de fromagerie doublée dans un bocal. On a noté des taux faibles de coagulum autour du thermocouple et de la pale d'agitation.

### 2) Préparation de compositions de coiffage :

Deux compositions de coiffage selon l'invention sous forme de shampooing ont été préparées en utilisant les composants ci-après dont les quantités sont exprimées en % en poids en l'état.

### Formulation A :

| | |
|---|---|
| Copolymère acrylate de 2-éthylhexyle (EMA) / acrylate de n-butyle (BA) / méthacrylate de 2-hydroxyéthyle (HEMA) 60/35/5 à 44,5% dans l'eau (CPC-00960-ACP45 Acrylates Copolymer de 3M) | 0,7 % |
| Chlorure de poly-diméthyldiallylammonium dans l'eau à 40 % non stabilisé (MERQUAT 100 de Nalco) | 0,25 % |
| Polymère acrylique à 30% en émulsion (AQUA SF1 de Noveon) | 3 % |
| Hydroxyde de sodium pur | 0,31 % |
| Chlorure de sodium | 2 % |
| Lauryléthersulfate de sodium (2,2 OE) à 70% en solution aqueuse | 14,11 % |
| Acide lauryléthercarboxylique (4,5 OE) à 90% en solution aqueuse | 2 % |
| Cocoyl bétaïne à 30% en solution aqueuse | 5,2 % |
| N-cocoyl amidoéthyl, N-éthoxycarboxyméthyl glycinate de sodium à 31% dans l'eau (MIRANOL CON2. de Rhodia) | 1 % |
| Myristate d'isopropyle | 1 % |
| Distéarate d'éthylène glycol | 1 % |
| Hexylène glycol (2 méthyl-2,4-pentanediol) | 1 % |
| Parfum, conservateurs | qsp |
| Eau désionisée microbiologiquement propre | qsp 100 % |

La formulation A utilisée sous forme de shampooing, c'est-à-dire appliquée sur les cheveux, puis lavée et rincée, confère aux cheveux une bonne facilité de coiffage et un effet repositionnable. On obtient des cheveux lisses qui présentent de bonnes propriétés de démêlage, de douceur et de brillance.

### Formulation B :

| | |
|---|---|
| Copolymère acrylate de 2-éthylhexyle (EMA) / acrylate de n-butyle (BA) / méthacrylate de 2-hydroxyéthyle (HEMA) 60/35/5 à 44,5% dans l'eau (CPC-00960-ACP45 Acrylates Copolymer de 3M) | 0,7 % |
| Chlorure de poly-diméthyldiallylammonium dans l'eau à 40 % non stabilisé (MERQUAT 100 de Nalco) | 0,25 % |
| Polymère acrylique à 30% en émulsion (AQUASF1 de Noveon) | 3 % |
| Hydroxyde de sodium pur | 0,31 % |
| Chlorure de sodium | 2 % |
| Lauryléthersulfate de sodium (2,2 OE) à 70% en solution aqueuse | 14,11 % |
| Acide lauryléthercarboxylique (4,5 OE) à 90% en solution aqueuse (vendu sous la dénomination AKYPORLM45 de Kao) | 2 % |
| Cocoyl bétaïne à 30% en solution aqueuse | 5,2 % |
| N-cocoyl amidoéthyl, N-éthoxycarboxyméthyl glycinate de sodium à 31% dans l'eau (MIRANOL CON2. de Rhodia) | 1 % |
| Huile d'avocat | 1 % |
| Distéarate d'éthylène glycol | 1 % |
| Hexylène glycol (2 méthyl-2,4-pentanediol) | 1 % |
| Parfum, conservateurs | qsp |
| Eau désionisée microbiologiquement propre | qsp 100 % |

La formulation B utilisée sous forme de shampooing, c'est-à-dire appliquée sur les cheveux, puis lavée et rincée, confère aux cheveux une bonne facilité de coiffage et un effet repositionnable. On obtient des cheveux lisses qui présentent de bonnes propriétés de démêlage, de douceur et de brillance.

## Revendications

1. Composition de coiffage repositionnable **caractérisée par le fait qu'**elle comprend, dans un véhicule cosmétiquement acceptable, un ou plusieurs copolymères (méth)acryliques comprenant
(a) des motifs issus d'un ou plusieurs monomères choisis parmi les monomères de (méth)acrylate de butyle, le ou les monomères (a) représentant de 15 à 50 % en poids par rapport à la quantité totale de monomères,
(b) des motifs issus d'un ou plusieurs monomères choisis parmi les monomères de (méth)acrylate d'hydroxyalkyle, et
(c) des motifs éventuels issus d'un ou plusieurs monomères copolymérisables autres que les monomères (a) et (b), et une ou plusieurs huiles non-siliconées.

2. Composition selon la revendication 1, **caractérisée en ce qu'**elle est rincée.

3. Composition selon la revendication 1 ou 2,
**caractérisée en ce que** l'huile non-siliconée est choisie parmi les huiles hydrocarbonées et/ou fluorées ou leurs mélanges.

4. Composition selon l'une des revendications 1 à 3, **caractérisée en ce que** l'huile hydrocarbonée est choisie parmi les huiles hydrocarbonée d'origine végétale et les esters liquides de synthèse.

5. Composition selon l'une des revendications 1 à 4, **caractérisée en ce que** le monomère cité dans (a) est choisi parmi les (méth)acrylates de n-butyle, les (méth)acrylates d'isobutyle, les (méth)acrylates de t-butyle et les (méth)acrylates de 2-méthylbutyle, de préférence l'acrylate de n-butyle.

6. Composition selon l'une quelconque des revendications 1 à 5, **caractérisée en ce que** le monomère cité dans (b) est choisi parmi les (méth)acrylates de 2-hydroxyéthyle et les (méth)acrylates d'hydroxypropyle, et de préférence le méthacrylate de 2-hydroxyéthyle.

7. Composition selon l'une quelconque des revendications 1 à 6, **caractérisée en ce que** le monomère copolymérisable cité dans (c) est choisi parmi (i') les monomères de (méth)acrylate d'alkyle, (ii') les monomères polaires, et (iii') les monomères polymérisables par voie radicalaire libre à insaturation éthylénique.

8. Composition selon la revendication 7, **caractérisée en ce que** les monomères de (méth)acrylate d'alkyle (i') sont choisis parmi les (méth)acrylates de méthyle, les (méth)acrylates d'isobornyle, les (méth)acrylates d'éthyle, le (méth)acrylates d'iso-octyle et les (méth)acrylates de 2-éthylhexyle, et de préférence l'acrylate de 2-éthylhexyle.

9. Composition selon la revendication 7, **caractérisée en ce que** les monomères polaires (ii') sont choisis parmi les acides (méth)acryliques, les acides itaconiques, les N-vinylpyrrolidones, les N-vinylcaprolactames, les (méth)acrylamides, les (méth)acrylates de diméthylaminoéthyle, les acrylonitriles, les (méth)acrylates de 2-carboxyéthyle, les anhydrides maléiques, et les monoacrylates de méthoxypolyéthylèneglycol 550.

10. Composition selon la revendication 7, **caractérisée en ce que** les monomères polymérisables par voie radicalaire libre à insaturation éthylénique (iii') sont choisis parmi le styrène et les esters de vinyle en C₁-C₄.

11. Composition selon l'une quelconque des revendications précédentes, **caractérisée en ce que** le copolymère (méth)acrylique (i) comprend :
- (a) de 30 à 40 % en poids de motifs issus d'un ou plusieurs monomères choisis parmi les monomères d'acrylate de n-butyle,
- (b) de 2 à 10 % en poids de motifs issus d'un ou plusieurs monomères choisis parmi les monomères de méthacrylate de 2-hydroxyéthyle, et
- (c) de 50 à 70 % en poids de motifs issus d'un monomère d'acrylate de 2-éthylhexyle.

12. Composition selon l'une quelconque des revendications 1 à 11, **caractérisée par le fait qu'**elle comprend en outre un ou plusieurs tensioactifs choisis parmi les tensioactifs anioniques, cationiques, amphotères, non-ioniques et leurs mélanges.

13. Composition selon l'une quelconque des revendications 1 à 12, **caractérisée par le fait qu'**elle comprend en outre un ou plusieurs agents conditionneurs choisis parmi les polymères non-siliconés cationiques, non-ioniques, amphotères ou les silicones.

14. Procédé de traitement cosmétique des cheveux, comprenant l'application sur les cheveux, avant leur mise en forme, d'une composition selon l'une quelconque des revendications 1 à 13.

15. Procédé de traitement des cheveux, comprenant :
(1) l'application sur les cheveux, avant la mise en forme initiale de la coiffure, d'une composition selon l'une quelconque des revendications 1 à 13, puis
(2) la mise en forme de la coiffure au moins une fois, **caractérisé en ce qu'**une composition supplémentaire ou de la chaleur supplémentaire n'est pas nécessaire.
